(19) Europäisches Patentamt
European Patent Office
Office européen des brevets

(11) Publication number : 0 434 621 A2

# (12) EUROPEAN PATENT APPLICATION

(21) Application number : 90810980.4

(22) Date of filing : 12.12.90

(51) Int. Cl.[5] : C07D 498/18, A61K 31/42, // (C07D498/18, 323:00, 307:00, 263:00)

(30) Priority : 21.12.89 US 454325
23.02.90 US 485345
31.08.90 US 576631

(43) Date of publication of application :
26.06.91 Bulletin 91/26

(84) Designated Contracting States :
AT BE CH DE DK ES FR GB GR IT LI LU NL SE

(71) Applicant : CIBA-GEIGY AG
Klybeckstrasse 141
CH-4002 Basel (CH)

(72) Inventor : Veenstra, Siem J., Dr.
Laufenstrasse 29
CH-4053 Basle (CH)
Inventor : Francis, John E., Dr.
15 Wexford Way
Basking Ridge, NJ 07920 (CH)
Inventor : Chen, Jen, Dr.
312 Clinton Avenue
Middlesex, NJ 08846 (US)

## (54) Rifamycin derivatives and processes for the manufacture thereof.

(57) Described are novel acyl derivatives of rifamycins of the formula

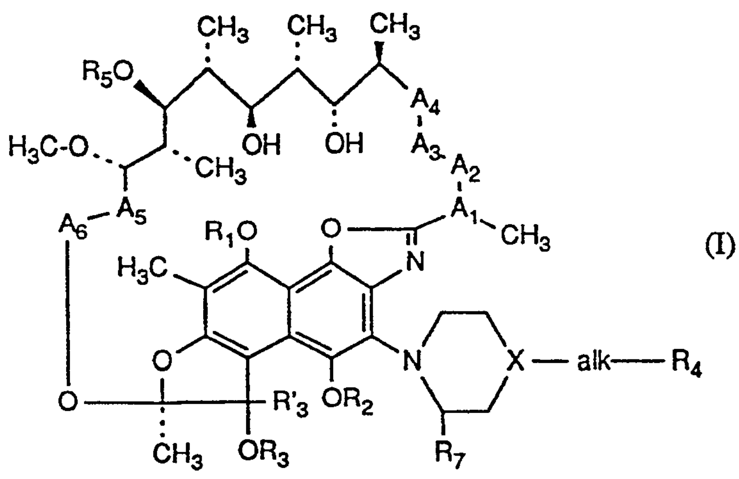

(I)

and the salts thereof, in which the structural elements $-A_1-A_2-$, $-A_3-A_4-$ and $-A_5-A_5-$ each denote ethylene or vinylene or the elements $-A_1-A_2-$ and $-A_3-A_4-$ each denote ethylene and $-A_5-A_6-$ denotes vinylene; X represents $>C(R_6)-$ or $>N-$ and $R_6$ denotes hydrogen or alkyl ; alk denotes an aliphatic hydrocarbon radical ; $R_1$ denotes hydrogen or acyl ; $R_2$ denotes alkyl which is optionally substituted by an aromatic radical, and $R_3$ and $R_3'$ represent a common bond, or $R_3$ denotes hydrogen or acyl, and $R_3'$ is hydrogen ; $R_4$ denotes hydrogen, cycloalkyl or aryl ; $R_5$ denotes hydrogen or acetyl ; and $R_7$ denotes hydrogen or alkyl, and processes for their manufacture. Said derivatives have hypolipidaemic activity and can be used as medicaments.

EP 0 434 621 A2

## RIFAMYCIN DERIVATIVES AND PROCESSES FOR THE MANUFACTURE THEREOF

The invention relates to novel acyl derivatives of rifamycins SV of the formula

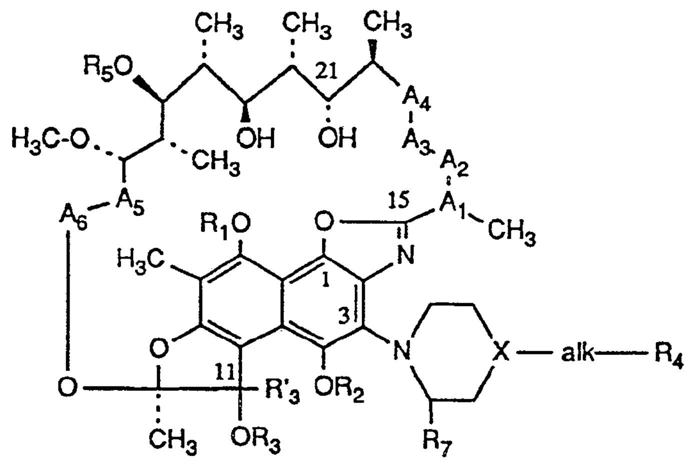

(I)

and the salts thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene; X represents $>C(R_6)-$ or $>N-$ and $R_6$ denotes hydrogen or alkyl ; alk denotes an aliphatic hydrocarbon radical ; $R_1$ denotes hydrogen or acyl ; $R_2$ denotes alkyl which is optionally substituted by an aromatic radical ; $R_3$ and $R_3'$ represent a common bond, or $R_3$ denotes hydrogen or acyl, and $R_3'$ is hydrogen ; $R_4$ denotes hydrogen, cycloalkyl or aryl ; $R_5$ denotes hydrogen or acetyl; $R_7$ denotes hydrogen or alkyl ; the preparation and use thereof, and pharmaceutical products and the preparation thereof.

The ring system numbering used as a basis essentially corresponds to that employed, for example, in US Patent No. 4,005,077.

The compounds of the formula I have several centres of chirality. The configurations at most C-atoms correspond to the known configurations of rifamycin S and rifamycin SV [cf. S.J. Danishefsky et al., J. Am. Chem. Soc. 109 (1987) 862-867]. If $-A_1-A_2-$ represents ethylene there is a further asymmetric centre at C-16. The methyl group bonded to C-16 is preferably in the α-position, i.e. below the plane of the paper like e.g. the 21-hydroxy group. If $R_3$ denotes hydrogen or acyl, and $R'_3$ is hydrogen, there is an additional asymmetric centre at C-11. In this case $R'_3$ may be in α- or β-position, but is preferably in α-position. Accordingly the present invention embraces the corresponding optical isomers, for example diastereoisomers.

The compounds of the formula I can be in the form of salts, especially pharmaceutically utilizable salts. Because the compounds according to the invention have at least one basic centre, they are therefore able to form acid addition salts. The latter are formed, for example, with inorganic acids such as mineral acids, for example sulfuric acid, a phosphorus or hydrohalic acid, or with organic carboxylic acids such as optionally substituted, for example by halogen, $C_1$-$C_4$alkanecarboxylic acids, for example acetic acid, such as optionally unsaturated dicarboxylic acids, for example oxalic, malonic, succinic, maleic, fumaric, phthalic or terephthalic acid, such as hydroxy carboxylic acids, for example ascorbic, glycolic, lactic, mallic, tartaric or citric acid, such as amino acids, for example aspartic or glutamic acid, with a benzoic acid or with organic sulfonic acids such as optionally substituted, for example by halogen, $C_1$-$C_4$alkane- or arylsulfonic acids, for example methane-, bromobenzene- or p-toluenesulfonic acid. Appropriate acid addition salts can also be formed with an additional basic centre which is present where appropriate (for example X = $>N-$ ). Furthermore, the compounds according to the invention which have an acidic phenolic hydroxyl group can form salts ($R_1$=H) with bases, for example alkali metals, such as sodium or potassium salts. In addition, corresponding inner salts can be formed. Also embraced are salts unsuitable for pharmaceutical uses, because the latter can be employed, for example, for the isolation or purification of compounds of the formula I according to the invention or the pharmaceutically utilizable salts thereof.

An aliphatic hydrocarbon radical denotes, in particular, alkylene, alkenylene and alkynylene, where the multiple bond is located in a position higher than α to the piperazine

nitrogen atom (X = 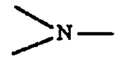 ).

Acyl is derived, for example, from an organic carboxylic acid, a substituted carbonic acid or an organic sulfonic acid. Examples of such radicals are alkanoyl, halogenoalkanoyl, arylalkanoyl, carbocyclic or heterocyclic aroyl, alkoxycarbonyl, arylalkoxycarbonyl, amino-carbonyl which is optionally mono- or disubstituted by alkyl, or alkanesulfonyl, halogenoalkanesulfonyl, arylalkanesulfonyl, cycloalkanesulfonyl or arylsulfonyl. In this connection, aryl denotes in each case, in particular, phenyl or naphthyl and carbocyclic aroyl, in particular benzoyl or naphthoyl and heterocyclic aroyl, in particular 5- or 6-membered monocyclic monoaza-, monooxa- or monothiaaroyl, such as pyrroloyl, pyridoyl, furoyl or thenoyl, where such aryl or aroyl radicals are unsubstituted or substituted one or more times, for example two or three times, for example by substituents selected from the group consisting of halogen, alkyl, alkoxy, hydroxyl, alkanoyloxy, trifluoromethyl and nitro.

An aromatic radical is, in particular, phenyl, furthermore naphthyl, which are unsubstituted or substituted one or more times, for example as indicated hereinafter for aryl. A corresponding alkyl substituted by an aromatic radical denotes, for example, phenyl- or naphthyl-($C_1$-$C_7$-)alkyl such as benzyl or 1- or 2-phenylethyl.

Aryl $R_4$ is derived, for example, from a mono- or polycyclic, such as bicyclic, C ring system which has at least one aromatic ring, such as phenyl, biphenylyl such as 2-, 3- or, in particular 4-biphenylyl, or naphthyl such as 1- or 2-naphthyl, and is unsubstituted or substituted, for example one or more times, such as two or three times, for example by substituents selected from the group consisting of halogen, ($C_1$-$C_7$-)alkyl, ($C_1$-$C_7$-)alkoxy, hydroxyl, ($C_2$-$C_8$-)alkanoyloxy, trifluoromethyl and nitro.

The general definitions used hereinbefore and hereinafter have primarily the following meanings, unless defined differently :

Alkyl denotes, in particular, $C_1$-$C_7$alkyl and is, for example, methyl, ethyl, propyl, isopropyl, n-butyl, isobutyl, sec-butyl, tert-butyl and furthermore embraces appropriate pentyl, hexyl and heptyl radicals. $C_1$-$C_4$Alkyl is preferred.

Cycloalkyl denotes, in particular, $C_3$-$C_7$cycloalkyl and denotes, for example, cyclopropyl, -butyl, -pentyl, -hexyl or -heptyl. Cyclopentyl and cyclohexyl is preferred.

Alkylene denotes, in particular, $C_1$-$C_7$alkylene, preferably $C_1$-$C_5$-alkylene, and is straight-chain or branched and denotes, for example, methylene, ethylene, propylene and butylene as well as 1,2-propylene, 2-methyl-1,3-propylene or 2,2-dimethyl-1,3-propylene. $C_1$-$C_4$Alkylene is preferred, primarily methylene.

Alkenylene denotes, in particular, $C_3$-$C_7$alkenylene and is straight-chain or branched and denotes, for example, 1,3-prop-2-enylene, 1,4-but-2-, 1,4-but-3-enylene, 1,3-but-2-enylene, 2,4-but-3-enylene, 1,5-pent-2-, -3-, -4-enylene, furthermore appropriate hexenylene and heptenylene radicals. $C_3$-$C_5$Alkenylene is preferred.

Alkynylene denotes, in particular, $C_3$-$C_7$alkynylene and is straight-chain or branched and denotes, for example, 1,3-prop-2-ynylene, 1,4-but-2-, 1,4-but-3-ynylene, 1,5-pent-2-, -3- and -5-ynylene, furthermore appropriate hexynylene and heptynylene radicals. $C_3$-$C_5$Alkynylene is preferred.

Alkanoyl denotes, in particular, $C_2$-$C_8$alkanoyl and is, for example, acetyl, propionyl, butyryl, isobutyryl or pivaloyl. Preferred, especially for $R_1$, is branched $C_2$-$C_6$alkanoyl, primarily pivaloyl, whereas $R_2$ and $R_3$ in particular are represented by $C_2$-$C_6$alkanoyl, primarily acetyl or propionyl.

Halogen is, in particular, halogen with atomic number up to and including 35, such as fluorine, chlorine and bromine, furthermore iodine.

Alkoxy denotes, in particular, $C_1$-$C_7$alkoxy and is, for example, methoxy, ethoxy, propyloxy, isopropyloxy, n-butyloxy, isobutyloxy and tert-butyloxy. $C_1$-$C_4$Alkoxy is preferred.

Alkoxycarbonyl is, in particular, $C_2$-$C_8$alkoxycarbonyl and is, for example, methoxy-, ethoxy-, propyloxy- or pivaloyloxy-carbonyl. $C_2$-$C_5$Alkoxycarbonyl is preferred.

Alkanesulphonyl is, in particular, $C_1$-$C_7$alkanesulfinyl or -sulfonyl and is, for example, methane-, ethane-, n-propane- or isopropane-sulfinyl or -sulfonyl. $C_1$-$C_4$Alkanesulfinyl or -sulfonyl is preferred.

Halogenalkanesulphonyl is, in particular, halo-$C_1$-$C_7$alkanesulphonoyl, in particular halo-$C_1$-$C_4$alkylsulphonoyl, and is, for example, trifluoromethane-, difluoromethane- or 1,1,2-trifluoroethanesulphonoyl.

Naphthyl is 1- or 2-naphthyl.

Pyrroloyl is, for example, 2- or 3-pyrroloyl. Furoyl is 2- or 3-furoyl and thenoyl is 2- or 3-thenoyl, while suitable pyridoyl is 2-, 3- and 4-pyridoyl.

Alkanoyloxy is, in particular, $C_2$-$C_8$alkanoyloxy, in particular, $C_2$-$C_5$alkanoyloxy, such as acetyloxy, propionyloxy or pivaloyloxy.

It is known of derivatives which are derived, for example, from rifamycin SV that they have pronounced antibiotic properties and can be employed, for example, for the treatment of tuberculosis. All the more important

is the experimentally verified finding that the compounds of the formula I and the pharmaceutically utilizable salts thereof show no corresponding antibiotic activity in the customary pharmacological test models.

On the other hand, surprisingly they have a significant lipid-lowering action which can be detected in animal experiments, preferably on mammals, for example on rats. Thus, the lowering of very low density, low density and high density lipoproteins (VLDL, LDL and HDL respectively) in the serum can be shown in two designs of test, namely in genetically hypercholesterolaemic male rats (design A) and normolipaemic rats of both sexes (design B).

Albino rats (Sprague-Dawley derivatives of the strain Tif : RAI) with a body weight of 180-240 g, which have free access to standard rat feed and drinking water, are used. The test compound is administered in a supplemented maize starch solution (3% aqueous maize starch with 0.33 % Tween 80 and 5 % polyethylene glycol solution with a mean molecular weight of 400) orally to groups of 6 rats each day for 5 consecutive days. The animals are sacrificed two hours after the last dose. The animals receive no more food for 16 hours before their death. The blood is collected in a 0.05 % strength aqueous ethylenediaminetetraacetic acid solution. After centrifugation until the blood cells have sedimented, the content of cholesterol and triglycerides is analysed enzymatically using, for example, the test systems supplied by Sigma Chemical Co. (St. Louis, MO, USA). For the HDL-cholesterol determination, a solution of heparin and manganese chloride (final concentration = 1.3 g/l or 46 mMol) is added to 0.5 ml of EDTA-plasma. The precipitate which forms is sedimented by centrifugation, and the cholesterol concentration in the supernatant is analysed enzymatically as for the total cholesterol. The difference between the latter value and the cholesterol value determined directly on an aliquot of the complete serum is, according to Warnick, G.R. et al., J. Lipid Res. 19 ; 65-76 (1978), equivalent to the VLDL- and LDL-cholesterol.

The testing for an antibiotic reaction is carried out, for example, on the one hand in vitro by determining the mean effective concentration $EC_{50}$ for the inhibition of RNA polymerase of Escherichia coli, and the minimum inhibitory concentration (MIC) in a conventional plate test, and on the other hand in vivo on infected mice and rats by determining the $ED_{50}$ (effective dose which keeps 50 % of the experimental animals alive). The microorganisms used for the present purpose are, in particular, Mycobacterium tuberculosis TB $H_{37}Rv$ and Staphylococcus aureus. In the case of compounds with a lipid-lowering indication, an antibiotic activity is regarded as disadvantageous because it may lead, especially on long-term administration, to the development of strains of microorganisms resistant to antibiotics.

In the test methods described above, the compounds according to the invention display a significant hypolipidaemic activity on repeated administration in the dose range from about 0.1 to about 50 mg/kg/day ; by contrast, they have practically no antibiotic activity in the abovementioned tests.

Thus, for example, it is possible to show, depending on the experimental designs, that the minimum effective dose of the compounds according to the invention is about 0.1 to about 10 mg/kg on single administration, and a 50-70 % lowering of total cholesterol can be achieved by repeated administration of 30 mg/kg a day. Moreover, the compounds have virtually no antibiotic activity ; an $EC_{50}$ for inhibition of RNA polymerase is not yet reached at 100 µg/ml, and the MIC for various pathogenic strains of Staphylococcus aureus is above 130 µg/ml. Such values are about 1000 times higher than concentrations normally necessary for a corresponding effect. Also in vivo, using mice infected with Staphylococcus aureus, the compound proves to have no antibiotic activity at a single dose of 200 mg/kg.

Thanks to their LDL-lowering action, the compounds according to the invention can be used, for example, as hypolipidaemics for the treatment of hyperlipidaemias, principally of types IIa and IIb, and arteriosclerosis, for example when hyperlipoproteinaemia is present as risk factor.

Accordingly, the compounds of the formula I and the pharmaceutically utilizable salts thereof can be used, for example, as pharmaceuticals, for example as hypolipidaemics for the treatement of hyperlipidaemias, principally of types IIa and IIb, and of arteriosclerosis when hyperlipoproteinaemia is present as risk factor. The invention furthermore relates to the use of the compounds according to the invention for the preparation of medicaments, in particular of hypolipidaemics and antiarteriosclerotics, and for therapeutic and prophylactic treatment. Also included therein is the industrial preparation of the active substances.

In opposite to prior art compounds corresponding to formula I in which $R_3$ and $R_3'$ together represent a double bond (and wherein $R_2$ is different from alkyl which is optionally substituted by an aromatic radical), those compounds of the present invention in which $R_3$ and $R_3'$ do not represent a double bond are surprisingly essentially colorless.

Furthermore, compounds of the formula I in which $R_2$ is different from hydrogen are more stable than those compounds corresponding to formula I in which $R_2$ represents hydrogen. The compounds of the present invention can additionally be used as starting material.

Preferred are compounds of the formula I and salts thereof, in which $-A_1-A_2-$, $-A_3-A_4-$,

$-A_5-A_6-$ and $R_5$ have the indicated meanings; X represents $>C(R_6)-$ or $>N-$, and $R_6$ denotes hydrogen or $C_1$-$C_7$alkyl ; alk denotes $C_1$-$C_7$alkylene, $C_3$-$C_7$alkenylene or $C_3$-$C_7$alkynylene ; $R_1$ denote $C_2$-$C_8$alkanoyl, halogen-$C_2$-$C_8$alkanoyl, phenyl- or naphthyl- $C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloal kanesulfonyl or benzene- or naphthylsulfonyl ; $R_2$ denotes $C_1$-$C_7$alkyl, phenyl- or naphthyl-$C_1$-$C_7$alkyl, $R_3$ and $R_3'$ represent a common bond, or $R_3$ denotes hydrogen or $C_2$-$C_8$alkanoyl, halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkylsulfonyl or benzene- or naphthylsulfonyl, and $R_3'$ is hydrogen; $R_4$ denotes hydrogen, $C_3$-$C_7$cycloalkyl, phenyl, biphenylyl or naphthyl ; $R_7$ denotes hydrogen or $C_1$-$C_7$alkyl ; where the aromatic radicals in each case are unsubstituted or substituted one or more times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro.

Especially preferred are compounds of the formula I and salts thereof, in which $-A_1-A_2-$, $-A_3-A_4-$, $-A_5-A_6-$ and $R_5$ have the indicated meanings; X represents $>C(R_6)-$ or $>N-$, and $R_6$ is hydrogen; alk denotes $C_1$-$C_7$alkylene, $C_3$-$C_7$alkenylene or $C_3$-$C_7$alkynylene ; $R_1$ and $R_3$, independently of one another, denote $C_2$-$C_8$alkanoyl, halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl, and $R_1$ additionally and $R_3'$ and $R_7$ denote hydrogen ; $R_2$ denotes $C_1$-$C_7$-alkyl, phenyl- or naphthyl-$C_1$-$C_7$alkyl ; $R_4$ denotes hydrogen, $C_3$-$C_7$cycloalkyl, phenyl, biphenylyl or naphthyl ; where the aromatic radicals are in each case unsubstituted or substituted one or more times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro.

Especially preferred are also compounds of the formula I and salts thereof, in which $-A_1-A_2-$, $-A_3-A_4-$, $-A_5-A_6-$ and $R_5$ have the indicated meanings ; X represents $>C(R_6)-$ or $>N-$, and $R_6$ is hydrogen; alk denotes $C_1$-$C_7$alkylene, $C_3$-$C_7$alkenylene or $C_3$-$C_7$alkynylene, in particular $C_1$-$C_4$alkylene, $C_3$-$C_4$alkenylene or $C_3$-$C_4$alkynylene, where the multiple bonds are located in a position higher than $\alpha$ to the piperazine nitrogen ; $R_1$ denotes $C_2$-$C_8$alkanoyl, in particular $C_2$-$C_6$alkanoyl, phenyl-$C_2$-$C_6$alkanoyl, benzoyl, $C_1$-$C_7$alkoxy-carbonyl such as $C_1$-$C_4$alkoxycarbonyl, phenyl-$C_1$-$C_4$alkoxycarbonyl, $C_1$-$C_4$alkanesulfonyl or benzenesulfonyl ; $R_2$ denotes $C_1$-$C_7$alkyl or phenyl-$C_1$-$C_7$alkyl ; $R_3$ and $R_3'$ together represent a bond or $R_3$ denotes hydrogen, $C_2$-$C_8$alkanoyl, in particular $C_2$-$C_6$alkanoyl, phenyl-$C_2$-$C_6$alkanoyl, benzoyl, $C_1$-$C_7$alkoxy-carbonyl such as $C_1$-$C_4$alkoxycarbonyl, phenyl-$C_1$-$C_4$alkoxycarbonyl, $C_1$-$C_4$alkanesulfonyl or benzenesulfonyl, and $R_3'$ is hydrogen ; $R_4$ denotes hydrogen, $C_3$-$C_7$cycloalkyl, phenyl, biphenylyl or naphthyl ; $R_7$ denotes hydrogen or $C_1$-$C_7$alkyl ; where the aromatic radicals in each case are unsubstituted or substituted up to three times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro.

More especially preferred are compounds of the formula I and salts thereof, in which $-A_1-A_2-$, $-A_3-A_4-$, $-A_5-A_6-$ have the indicated meanings; X represents $>C(R_6)-$ or $>N-$, and $R_6$ is hydrogen; alk denotes $C_1$-$C_7$alkylene, in particular $C_1$-$C_4$alkylene;

$R_1$ denotes $C_2$-$C_8$alkanoyl, in particular $C_2$-$C_6$alkanoyl ; $R_2$ denotes $C_1$-$C_4$alkyl or phenyl-$C_1$-$C_4$alkyl, $R_3$ and $R_3'$ together represent a bond, or $R_3$ denotes hydrogen, $C_2$-$C_8$alkanoyl, in particular $C_2$-$C_6$alkanoyl, benzoyl, $C_1$-$C_4$alkanesulfonyl or benzenesulfonyl, and $R_3'$ is hydrogen ; $R_4$ denotes hydrogen, $C_3$-$C_7$cycloalkyl, phenyl, biphenylyl or naphthyl, $R_5$ is acetyl ; $R_7$ is hydrogen or $C_1$-$C_4$alkyl ; where the aromatic radicals in each case are unsubstituted or substituted up to three times by substituents selected from the group consisting of halogen, $C_1$-$C_4$alkyl, $C_1$-$C_4$alkoxy, hydroxyl, $C_2$-$C_6$alkanoyloxy, trifluoromethyl and nitro.

More especially preferred are also compounds of the formula I and salts thereof, in which

-$A_1$-$A_2$-, -$A_3$-$A_4$- and -$A_5$-$A_6$- have the indicated meanings; X represents $>C(R_6)—$ or $>N—$, and $R_6$ is hydrogen; alk denotes $C_1$-$C_5$alkylene such as methylene, 2-methyl-1,3-propylene or 3-methyl-1,4-butylene ; $R_1$ denotes branched $C_3$-$C_6$alkanoyl, in particular pivaloyl ; $R_2$ denotes $C_1$-$C_4$alkyl such as methyl or ethyl, phenyl-$C_1$-$C_4$alkyl such as benzyl or 2-phenyl-ethyl ; $R_3$ denotes hydrogen, $C_2$-$C_6$alkanoyl such as acetyl, propionyl or pivaloyl, benzoyl or $C_1$-$C_4$alkanesulfonyl such as methanesulfonyl, and $R_3'$ is hydrogen ; $R_4$ denotes hydrogen, $C_3$-$C_7$cycloalkyl such as cyclopentyl or -hexyl, phenyl which is optionally substituted by up to three $C_1$-$C_4$alkyl-substituents, in particular 2,4,6-trimethylphenyl; $R_5$ is acetyl ; $R_7$ denotes hydrogen or $C_1$-$C_4$alkyl such as methyl.

Primarily preferred are compounds of the formula I and salts thereof, in which the structural elements -$A_1$-$A_2$-, -$A_3$-$A_4$- and -$A_5$-$A_6$- each have the indicated meanings ; X represents $>N—$ ; $R_1$ denotes branched $C_3$-$C_6$alkanoyl, in particular pivaloyl; $R_2$ denotes $C_1$-$C_4$alkyl such as methyl or ethyl ; $R_3$ denotes hydrogen or $C_2$-$C_5$alkanoyl such as acetyl, furthermore propionyl or pivaloyl ; $R_3'$ is hydrogen ; $R_5$ is acetyl ; alk-$R_4$ denotes $C_3$-$C_5$alkyl in particular isobutyl or neopentyl, or $C_3$-$C_6$cycloalkyl-methyl in particular cyclopentylmethyl or cyclohexylmethyl, or 2,4,6-tri-$C_1$-$C_4$-alkyl-benzyl, in particular 2,4,6-trimethylbenzyl ; $R_7$ is hydrogen.

The invention primarily relates to compounds of the formula I and pharmaceutically acceptable salts thereof, in which the structural elements -$A_1$-$A_2$-, -$A_3$-$A_4$- and -$A_5$-$A_6$- each denote ethylene or vinylene or the elements -$A_1$-$A_2$- and -$A_3$-$A_4$- each denote ethylene and -$A_5$-$A_6$- denotes vinylene; X represents $>CH—$ or $>N—$ ; alk denotes methylene or 2,2-dimethyl-1,3-propylene ; $R_1$ denotes pivaloyl ; $R_2$ denotes methyl, ethyl or benzyl ; $R_3$ and $R_3'$ represent a common bond, or $R_3$ denotes hydrogen or acetyl, and $R_3'$ is hydrogen ; $R_4$ denotes hydrogen, cyclohexyl or 2,4,6-trimethyl-phenyl ; $R_5$ denotes hydrogen or acetyl ; $R_7$ denotes hydrogen.

The invention primarily relates to compounds of the formula I and salts thereof, in which the structural elements -$A_1$-$A_2$-, -$A_3$-$A_4$- and -$A_5$-$A_6$- each denote ethylene or vinylene or the elements -$A_1$-$A_2$- and -$A_3$-$A_4$- each denote ethylene and -$A_5$-$A_6$- denotes vinylene ; X represents $>N—$ ; $R_1$ denotes branched $C_3$-$C_5$alkanoyl, primarily pivaloyl; $R_2$ denotes $C_1$-$C_4$alkyl in particular methyl, furthermore ethyl ; $R_3$ and $R_3'$ each denote hydrogen ; $R_5$ denotes acetyl ; $R_7$ is hydrogen ; and alk-$R_4$ denotes 2,4,6-trimethylbenzyl.

The invention primarily relates to compounds of the formula I and salts thereof, in which the structural elements -$A_1$-$A_2$-, -$A_3$-$A_4$- and -$A_5$-$A_6$- each denote vinylene ; X represents $>N—$ ; $R_1$ denotes branched $C_3$-$C_5$alkanoyl, primarily pivaloyl; $R_2$ denotes $C_1$-$C_4$alkyl primarily methyl, furthermore ethyl ; $R_3$ and $R_3'$ each denote hydrogen ; $R_5$ denotes acetyl ; $R_7$ is hydrogen ; and alk-$R_4$ denotes 2,4,6-trimethylbenzyl.

Most preferred are the novel compounds, especially those of the formula I, mentioned in the examples, and the preparation thereof.

The invention likewise relates to processes for the preparation of the compounds according to the invention. The preparation of compounds of the formula I and salts thereof is carried out in a manner known per se and is characterized in that, for example,

a) for the preparation of compounds of the formula I and salts thereof, in which X

represents 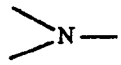, a rifamycin SV derivative of the formula

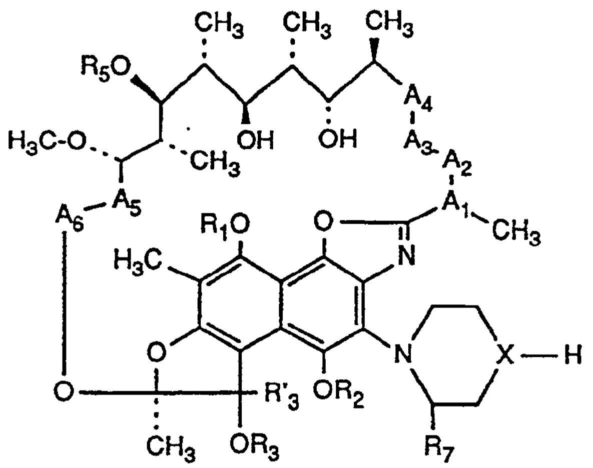

(IIa)

or a salt thereof, in which X represents 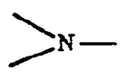 and the other symbols have the abovementioned meanings, is reacted with a compound of the formula

Z-alk-$R_4$ [IIb]

in which Z denotes reactive esterified hydroxyl and the other symbols have the abovementioned meanings, or

b) a rifamycin SV derivative of the formula

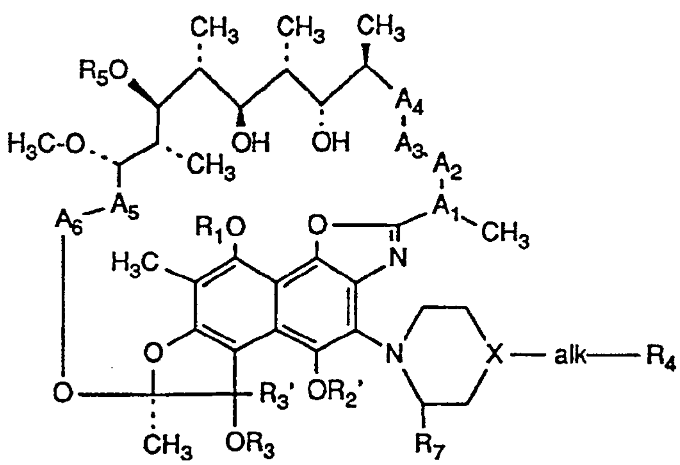

(III)

in which $R_2'$ is hydrogen or, if $R_3$ is hydrogen, $R_2'$ may also represent acyl, and the other symbols have the abovementioned meanings, is alkylated and/or a rifamycin SV derivative of the formula III in which at least one of the radicals $R_1$ and $R_3$ denotes hydrogen, $R_2'$ has the abovementioned meaning of $R_2$, and the other symbols have the abovementioned meanings, is acylated, or

c) a rifamycin SV derivative of the formula

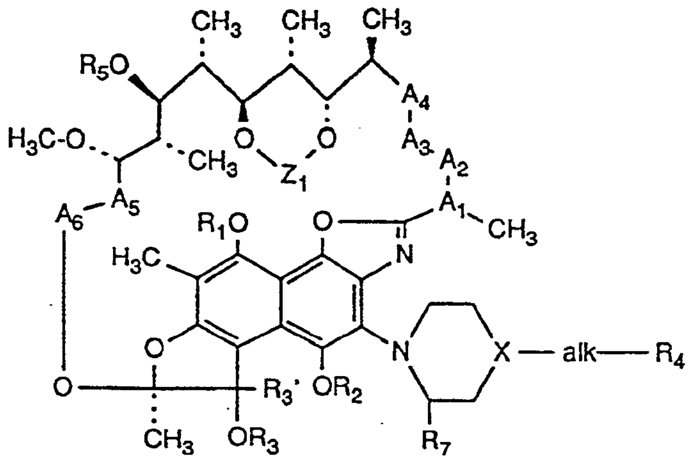

(IV)

in which $Z_1$ denotes alkylidene or cycloalkylidene and the other symbols have the abovementioned meanings, is hydrolysed, or

d) for the manufacture of compounds of the formula I wherein $R_1$ represents pivaloyl, a rifamycin SV derivative of the formula V,

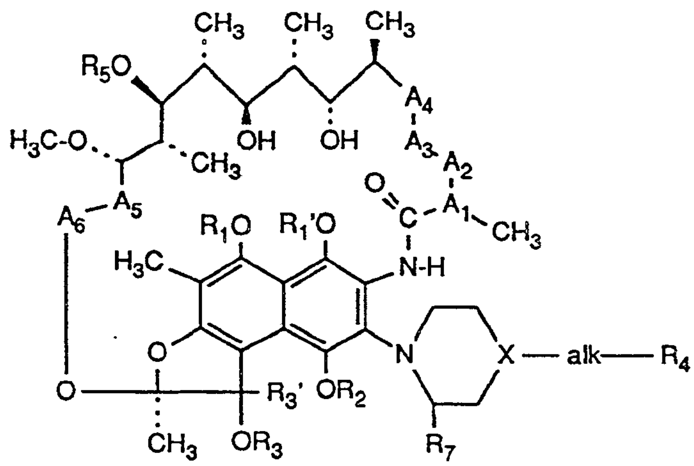

(V)

wherein $R_1$ represents pivaloyl and $R_1'$ represents pivaloyl or hydrogen and the remaining symbols have the meanings given above, is heated until cyclisation has occurred, and, if desired, a compound of the formula I obtainable according to the process or by other means, or a salt thereof, is converted into another compound according to the invention, or a salt thereof ; a free compound of the formula I obtainable according to the process is converted into a salt ; or a salt obtainable according to the process is converted into the free compound of the formula I or into another salt ; and, if desired, a mixture of isomers obtainable according to the process is fractionated.

Salts of the starting materials of the formulae IIa, III and IV which have an acidic phenolic hydroxyl group are appropriate salts with bases of the type detailed hereinbefore, whereas the starting compounds of the formula IIa, III or IV which have one or two basic centres can form corresponding acid addition salts in analogy to the acid addition salts of the formula I.

Reactive esterified hydroxyl, for example Z, is, in particular, hydroxyl which is esterified with a strong inorganic acid or organic sulfonic acid, for example halogen such as chlorine, bromine or iodine, sulfonyloxy such as hydroxysulfonyloxy, halogenosulfonyloxy, for example fluorosulfonyloxy, optionally substituted, for example by halogen, $C_1$-$C_7$alkanesulfonyloxy, for example methane- or trifluoromethanesulfonyloxy, $C_5$-$C_7$cycloalkanesulfonyloxy, for example cyclohexanesulfonyloxy, or optionally substituted, for example by $C_1$-$C_7$alkyl or halogen, benzenesulfonyloxy, for example p-bromobenzene- or p-toluenesulfonyloxy.

Alkylidene denotes, for example, $C_1$-$C_7$alkylidene, in particular $C_1$-$C_5$alkylidene such as methylene, ethylidene, isopropylidene, 1-methyl-propylidene or -butylidene, whereas cycloalkylidene denotes, for example, $C_3$-$C_7$cycloalkylidene, in particular cyclopentylidene or -hexylidene.

If not herein expressly stated otherwise, the reactions described hereinbefore and hereinafter in the variants are carried out in a manner known per se, for example, in the absence or customary manner in the presence

of a suitable solvent or diluent or of a mixture thereof, and they are carried out, as required, with cooling, at room temperature or with heating, for example in a temperature range from about -80° up to the boiling point of the reaction medium, preferably from about -10° up to about+180°C and, if necessary, in a closed vessel, under pressure, in an inert gas atmosphere and/or under anhydrous conditions.

Variant a) : Z preferably denotes halogen, such as chlorine, bromine or iodine, furthermore sulfonyloxy such as methane- or p-toluenesulfonyloxy.

The reaction is carried out in a manner known per se, advantageously in the presence of a base.

Suitable and preferred bases are non-nucleophilic tertiary amines, for example tri-lower-alkylamines, basic heterocycles and carbocyclic amines such as ethyl-diisopropylamine, triethylamine, pyridine, 1,5-diaza-bicyclo[4.3.0]non-5-ene (DBN) and 1,8-diaza-bicyclo[5.4.0]undec-7-ene (DBU).

Variant b) : The acylation is carried out in a manner known per se using a suitable acylating agent. An example of a suitable acylating agent is a compound of the formula Ac-$Z_2$ (IIIa), where Ac denotes an acyl radical corresponding to the variables $R_1$, $R_2$ and $R_3$, and $Z_2$ denotes hydroxyl or, in particular, reactive activated hydroxyl. Appropriate hydroxyl can be activated, for example, by strong acids such as hydrohalic or carboxylic acid, for example by hydrochloric, hydrobromic acid, an optionally substituted, for example by halogen, alkanecarboxylic acid or by an acid of the formula Ac-OH, or by suitable activating or coupling reagents of the type detailed hereinafter, in particular in situ. Ac-$Z_2$ can furthermore represent an activated ester, where $Z_2$ denotes, in particular, cyanomethoxy, (4-)nitrophenoxy or polyhalogeno-, such as pentachloro-, -phenoxy. Activating and coupling reagents which can be employed are, in particular, carbodiimides, for example N,N'-di-$C_1$-$C_4$alkyl- or N,N'-di-$C_5$-$C_7$cycloalkyl-carbodiimide, such as diisopropylcarbodiimide or N,N'-dicyclohexylcarbodiimide, advantageously with the addition of an activating catalyst such as N-hydroxysuccinimide or optionally substituted, for example by halogen, $C_1$-$C_7$alkyl or $C_1$-$C_7$alkoxy, N-hydroxy-benzotriazole or N-hydroxy-5-norbornene-2,3-dicarboxamide, furthermore $C_1$-$C_4$alkyl halogenoformate, for example isobutyl chloroformate, suitable carbonyl compounds, for example N,N-carbonyldiimidazole, suitable 1,2-oxazolium compounds, for example 2-ethyl-5-phenyl-1,2-oxazolium 3'-sulfonate or 2-tert-butyl-5-methyl-isoxazolium perchlorate, suitable acylamino compounds, for example 2-ethoxy-1-ethoxycarbonyl-1,2-dihydroquinoline, or suitable phosphoryl cyanamides or azides, for example diethylphosphoryl cyanamide or diphenylphosphoryl azide, furthermore triphenylphosphine disulfide or 1-$C_1$-$C_4$alkyl-2-halogeno-pyridinium halides, for example 1-methyl-2-chloropyridinium iodide.

$Z_2$ preferably denotes halogen such as chlorine or bromine, and Ac-O-.

The acylation according to the invention is preferably carried out under mild conditions, for example at room temperature or slightly elevated temperatures. Depending on the nature of the starting material of the formula III, the amount of acylating agent must be selected so that one or, secondarily, two acyl groups are introduced. The acylating agent can advantageously act as solvent. The course of the reaction is expediently followed by customary analytical methods, in particular using thin-layer chromatography.

If the acylation is carried out with a compound of the formula Ac-$Z_2$ in which $Z_2$ denotes halogen, it is advantageously carried out in the presence of an acid-binding agent such as a base which cannot be acylated. Suitable bases are, for example, alkali metal hydroxides, hydrides, amides, alkanolates, carbonates, triphenylmethylides, di(lower alkyl)amides, aminoalkylamides or lower alkyl silylamides, or naphthaleneamines, lower alkylamines, basic heterocycles, ammonium hydroxides, and also carbocyclic amines. Examples which may be mentioned are sodium hydroxide, sodium hydride, sodium amide, sodium (m-)ethoxide, potassium tert-butoxide, cesium carbonate, potassium carbonate, lithium triphenylmethylide, lithium diisopropylamide, potassium 3- (aminopropyl)amide, potassiumbis(trimethylsilyl)amide, dimethylaminonaphthalene, di- or triethylamine, or ethyldiisopropylamine, N-methylpiperidine, pyridine, benzyltrimethylammonium hydroxide, 1,5-diazabicyclo[4.3.0]non-5-ene (DBN) and 1,8-diazabicyclo[5.4.0]undec-7-ene (DBU). In the reaction with an anhydride, in particular a symmetric anhydride, an excess of Ac-O-Ac is used, in particular.

The phenolic 4-hydroxyl group can be etherified in a manner known per se. The etherification can be carried out, for example, with a reactive ester of the alkanol which is optionally substituted by an aromatic radical. Examples of suitable reactive esters of the relevant alcohols are those with strong inorganic or organic acids, such as appropriate halides, sulfates, sulfonates, for example lower alkanesulfonates or optionally substituted benzenesulfonates, in particular chlorides, bromides, iodides, methane-, benzene- or p-toluenesulfonates. The etherification can be carried out, for example, in the presence of a base such as an alkali metal hydride, hydroxide, carbonate or of a basic amine. It is advantageous to start from an appropriate alkali metal salt of the formula III.

The preparation of compounds of the formula I in which $R_2$ denotes alkyl which is optionally substituted by an aromatic radical, and $R_3$ denotes acyl, primarily alkanoyl, can start from corresponding compounds of the formula III in which $R_2$ is acyl and $R_3$ denotes hydrogen, and the latter are alkylated in the indicated manner, preferably under basic conditions. This is because the 4-O-acyl group migrates to the 11-hydroxyl group.

If both $R_2'$ and $R_3$ denote hydrogen, under usual reaction conditions, the 4-OH group is preferably esterified or etherified, respectively, first.

The preparation of the starting material of the formula III starts, for example, from rifamycin S or SV and introduces the side-chain

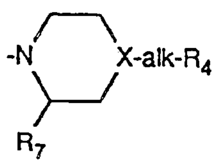

in position 3 in a manner known per se, for example as described in WO 87/02361. The appropriate SV derivative is subsequently converted using an acylating agent, such as pivaloyl chloride, into the corresponding 1,8-di-O-acyl compound and then, by prolonged heating, for example at 100°C, into the corresponding 8-O-acyl-1-deoxy-15-deoxo-1,15-oxy derivative of the formula III. Corresponding hydro compounds can be prepared by hydrogenation in a manner known per se, for example as described hereinafter.

Variant c) : $Z_1$ primarily represents $C_1$-$C_5$alkylidene such as isopropylidene. The ketal of the formula IV is hydrolysed in a manner known per se, advantageously using an acid such as an inorganic or organic acid, such as mineral acid, for example a hydrohalic acid, sulfuric acid or a phosphorus acid, sulfonic acid, for example methane- or p-toluenesulfonic acid, or a carboxylic acid, for example acetic acid.

The preparation of compounds of the formula IV is carried out in a manner known per se. Thus, for example, it starts from a compound of the formula I in which $R_2$ is hydrogen and $R_3$ and $R_3'$ together represent a bond or each denote hydrogen, and the latter is reacted in the presence of one of the listed acids with an aldehyde or ketone, or a ketal thereof, such as di-$C_1$-$C_4$alkyl or $C_2$-$C_4$alkylene ketal, for example acetone dimethyl ketal, corresponding to $Z_1$. In turn, compounds of the formula I in which $-A_1-A_2-$, $-A_3-A_4-$ and $-A_5-A_6-$ each denote vinylene, and $R_3$ and $R_3'$ together represent a bond, are described, for example, in European patent application publication no. 314624. Corresponding hydro derivatives ($-A_1-A_2-$, $-A_3-A_4-$ and $-A_5-A_6-$ denote ethylene, for example) can be obtained, for example, using the hydrogenation processes detailed hereinafter.

Variant d) : The treatment of compounds of formula V is carried out with heating, for example in a temperature range from about 50 to 180°C, preferably from about 100 to 170°C. The heating can also be achieved by microwave irradiation.

The starting material of formula V is prepared, for example, by reacting rifamycin S or 3-halo-rifamycin S, preferably 3-bromo-rifamycin S, with an amine of formula VI,

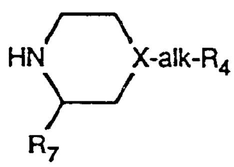

(VI)

wherein the symbols have the abovementioned meanings.

The reaction is carried out with an excess of the amine of formula VI, for example in a temperature range from about 0 to 100°C, whereupon a mixture of the quinone and hydroquinone form is obtained. This mixture ($R_1 = H$) is converted into the corresponding hydroquinone (derivative of rifamycin SV) by reduction, for example by catalytic hydrogenation. Afterwards compounds of formula V, wherein $R_1$ and/or $R_1'$ are pivaloyl, are obtained by treatment with suitable acylating agents, for example with an acid anhydride such as pivaloyl chloride, in the presence of a base, for example pyridine. Finally, the 4-hydroxy group is etherified in a manner known per se, e.g. as described hereinbefore for variant b).

A compound of the formula I obtainable according to the invention or in another manner, or salt thereof, can be converted in a manner known per se into another compound of the formula I.

Compounds of the formula I in which the structural elements $-A_1-A_2-$, $-A_3-A_4-$ and $-A_5-A_6-$ each denote vinylene or $-A_1-A_2-$ and $-A_3-A_4-$ each denote ethylene and $-A_5-A_6-$ denotes vinylene can be converted by reduction, for example by catalytic hydrogenation, into the corresponding tetrahydro-($-A_1-A_2-$ and $-A_3-A_4-$ are each ethylene and $-A_5-A_6-$ is vinylene) or the corresponding hexahydro derivatives ($-A_1-A_2-$, $-A_3-A_4-$ and $-A_5-A_6-$ each denote ethylene). Thus the hydrogenation of the multiple bonds takes place in the presence of hydrogenation catalysts, suitable for this purpose being, for example, noble metals or derivatives thereof, for examples oxides,

such as nickel, Raney nickel, palladium, platinum oxide, which can optionally be absorbed on support materials, for example on charcoal or calcium carbonate. The hydrogenation can be carried out, in particular, under pressures of 1 to about 100 at.

Acetyl $R_5$ can selectively be replaced by hydrogen in the presence of other acyl groups, for example, if $R_1$ denotes pivaloyl, by treatment with hydrazine (hydrate) or a suitable derivative thereof.

Compounds of the formula I in which, for example one of the radicals $R_1$ and $R_3$ denotes acyl, and the other radical is hydrogen, can be acylated in a manner known per se, for example in analogy to the methods described in variant b), for example by reaction with the appropriate carboxylic acid or with a reactive derivative thereof. Examples of reactive derivatives of this type are anhydrides, including mixed anhydrides, such as an acid halide, for example chloride, or anhydrides with a formic ester, activated carboxylic esters such as cyanomethyl, (4-)nitrophenyl, polyhalogenophenyl, for example pentachlorophenyl, esters. The reaction with the carboxylic acid or a salt thereof takes place under water-eliminating conditions, for example with azeotropic removal of the water of reaction, or by treatment with a suitable condensing agent, for example N,N'-dicyclohexyl-carbodiimide. The reaction with a reactive acid derivative is advantageously carried out in the presence of a base. Correspondingly, the acetyl radical $R_5$ can be introduced into compounds of the formula I in which $R_5$ is hydrogen by treatment with an appropriate acetylating agent.

It is possible by treatment with strong bases, such as alkali metal hydroxides, to replace the acetyl radical $R_5$ and the acyl radical $R_1$ and/or $R_3$ by hydrogen. The acyl radical $R_1$ can also be selectively eliminated in the presence of the acetyl radical $R_5$, for example by treatment with a fluoride such as alkali metal, for example sodium or cesium fluoride, or with an ammonium fluoride, for example tetrabutylammonium fluoride.

Salts of compounds of the formula (I) can be prepared in a manner known per se. Thus, for example, acid addition salts of compounds of the formula (I) are obtained by treatment with an acid or with a suitable ion exchanger reagent. Salts can be converted in a customary manner into the free compounds, acid addition salts, for example, by treatment with a suitable basic agent.

Depending on the procedure and reaction conditions, the compounds according to the invention with salt-forming, in particular basic, properties can be obtained in free form or, preferably, in the form of salts.

As a consequence of the close relationship between the novel compound in free form and in the form of salts thereof, hereinbefore and hereinafter by the free compound or salts thereof is meant, where appropriate for the sense and purpose, also the corresponding salts or the free compound.

The novel compounds, including their salts of salt-forming compounds, can also be obtained in the form of hydrates thereof, or include other solvents used for crystallization.

The novel compounds can, depending on the choice of starting materials and procedures, be in the form of one of the possible isomers or as mixtures thereof, for example depending on the number of asymmetric carbon atoms, as pure optical isomers, such as antipodes, or as mixtures of isomers, such as racemates, diastereoisomeric mixtures or racemate mixtures.

Racemate mixtures which are obtained can be separately fractionated on the basis of the physicochemical differences of the components in a known manner into the pure isomers or racemates, for example by fractional crystallization. Racemates which are obtained can, furthermore, be resolved by known methods into the optical antipodes, for example by recrystallization from an optically active solvent, chromatography on chiral adsorbents, using suitable microorganisms, by cleavage with specific, immobilized enzymes, via the formation of inclusion compounds, for example using chiral crown ethers, in which case only one enantiomer is complexed, or by conversion into diastereomeric salts, for example by reaction of a basic final compound racemate with an optically active acid such as carboxylic acid, for example tartaric or mallic acid, or sulfonic acid, for example camphorsulfonic acid, and separation of the diastereomeric mixture obtained in this manner, for example on the basis of their different solubilities, into the diastereomers, from which the desired enantiomer can be liberated by the action of suitable agents. The more active enantiomer is advantageously isolated.

The working up of the reaction product from the reaction mixture obtainable according to the process is carried out in a manner known per se, for example by dilution with water, and/or where appropriate by neutralization or slight acidification (to about pH = 3) with an aqueous acid, such as an inorganic or organic acid, for example a mineral acid or, advantageously, citric acid, and addition of a solvent which is immiscible with water, such as chlorinated hydrocarbon, for example chloroform or methylene chloride, in which case the reaction product transfers into the organic phase, from which it can be obtained in pure form in a customary manner, for example by drying, evaporation of the solvent and crystallization and/or chromatography of the residue or other customary purification methods. If the above reaction yields, for example, a mixture of acylated compounds, the latter can be fractionated in a manner known per se, for example by fractional crystallization, chromatography etc., into the desired individual acyl compounds.

The invention likewise relates to the novel compounds obtainable by the abovementioned process variants.

The invention also relates to those embodiments of the process which start from a compound obtainable

at any one stage of the process as intermediate, and the missing steps are carried out or a starting material is used in the form of a derivative or salt and/or its racemates or antipodes, or, in particular, is formed under the reaction conditions.

In the process of the present invention, the starting materials which are preferably used are those which lead to the compounds described as particularly valuable in the introduction. The invention likewise relates to novel starting materials which have been developed specifically for the preparation of the compounds according to the invention, in particular to novel compounds of the formulae III or V, the use thereof as hypolipidaemics and antibiotics, respectively, and to processes for the preparation thereof, where the variables $-A_1-A_2-$, $-A_3-A_4-$, $-A_5-A_6-$, $R_1$, $R_2$, $R_3$, $R_3'$, $R_4$, $R_5$, $R_6$, $R_7$, X and alk have the meanings indicated for the preferred groups of compounds of the formula I in each case.

The present invention also embraces the use of compounds of the formula I and of pharmaceutically utilizable, i.e. pharmaceutically acceptable salts thereof alone or together with auxiliaries, as well as in combination with other active compounds, as agents for the therapeutic treatment, namely both curative and preventive, of diseases or pathological states indicated or caused, for example, by an elevated content of cholesterol and/or triglycerides in the blood, in particular in blood serum. The active compounds according to the invention are administered in therapeutically effective amounts, preferably in the form of pharmaceutical compositions together with conventional pharmaceutical vehicles and/or auxiliaries, to the warm-blooded animals, primarily humans, requiring treatment. This entails, for example, administration to warm-blooded animals, depending on the species, body weight, age and individual condition, of daily doses corresponding to about 1 to about 100, in particular about 3 to about 50, mg per kg of body weight, which can be exceeded in severe cases. Accordingly, the invention also embraces the corresponding method for medical treatment.

The invention likewise relates to pharmaceutical products, e.g. pharmaceutical compositions, which contain the compounds according to the invention, or pharmaceutically utilizable salts thereof, as active compounds, and to processes for the preparation thereof.

The pharmaceutical products according to the invention which contain the compound according to the invention or pharmaceutically utilizable salts thereof are those for enteral, such as oral furthermore rectal, and parenteral administration to warm-blooded animal(s), the pharmacological active compound being contained alone or together with a pharmaceutically utilizable vehicle. The daily dosage of the active compound depends on the age and the individual condition as well as on the mode of administration.

The novel pharmaceutical products contain, for example, from about 10 % to about 80 %, preferably from about 20 % to about 60 %, of the active compound. Examples of pharmaceutical products according to the invention for enteral or parenteral administration are those in dose-unit forms such as coated tablets, tablets, capsules or suppositories, as well as ampoules. These are prepared in a manner known per se, for example using conventional mixing, granulating, coating, dissolving or freeze-drying processes. Thus, pharmaceutical products for oral use can be obtained by combining the active compound with solid excipients, where appropriate granulating a mixture which is obtained, and processing the mixture or granules, if desired or necessary, after addition of suitable auxiliaries to tablets or cores of coated tablets.

Particularly suitable excipients are fillers such as sugars, for example lactose, sucrose, mannitol or sorbitol, cellulose products and/or calcium phosphates, for example tricalcium phosphate or calcium hydrogen phosphate, as well as binders such as starch mucillage using, for example, maize, wheat, rice or potato starch, gelatin, tragacanth, methylcellulose and/or polyvinylpyrrolidone, if desired, disintegrants such as the abovementioned starches, as well as carboxymethyl starch, crosslinked polyvinylpyrrolidone, agar, alginic acid or a salt thereof, such as sodium alginate, auxiliaries are primarily flowability and flow-regulating agents and lubricants, for example silica, talc, stearic acid or salts thereof, such as magnesium or calcium stearate, and/or polyethylene glycol. Cores of coated tablets are provided with suitable, optionally enteric, coatings, using, inter alia, concentrated sugar solutions which optionally contain gum arabic, talc, polyvinylpyrrolidone, polyethylene glycol and/or titanium dioxide, lacquer solutions in suitable organic solvents or solvent mixtures or, for the preparation of enteric coatings, solutions of suitable cellulose products such as acetyl cellulose phthalate or hydroxypropylmethylcellulose phthalate. Colorants or pigments can be added to the tablets or coatings of coated tablets, for example, to identify or to indicate various doses of active compound.

Further pharmaceutical products which can be used orally are two-piece capsules made of gelatin, as well as soft, closed capsules made of gelatin and a plasticizer such as glycerol or sorbitol. The two-piece capsules can contain the active compound in the form of granules, for example mixed with fillers such as lactose, binders such as starches, and/or glidants such as talc or magnesium stearate, and, where appropriate, stabilizers. The active compound in soft capsules is preferably dissolved or suspended in suitable liquids such as fatty oils, liquid paraffin or liquid polyethylene glycols, it likewise being possible to add stabilizers.

Examples of pharmaceutical products suitable for rectal use are suppositories which consist of a combination of the active compound and a suppository base. Examples of suitable suppository bases are natural or

synthetic triglycerides, paraffin hydrocarbons, polyethylene glycols or higher alkanols. It is furthermore possible to use gelatin rectal capsules which contain a combination of the active compound with a base substance. Examples of suitable base substances are liquid triglycerides, polyethylene glycols or paraffin hydrocarbons.

Primarily suitable for parenteral administration are aqueous solutions of an active compound in water-soluble form, for example of a water-soluble salt, furthermore suspensions of the active compound such as appropriate oily injection suspensions, in which case suitable lipophilic solvents or vehicles such as fatty oils, for example sesame oil, or synthetic fatty acid esters, for example ethyl oleate or triglycerides, are used, or aqueous injection suspensions which contain viscosity-increasing substances, for example sodium carboxymethylcellulose, sorbitol and/or dextran and, where appropriate, also stabilizers.

The dosage of the active compound depends on the warm-blooded species, the age and the individual condition as well as the mode of administration. In the normal case for a warm-blooded animal weighing about 75 kg the estimated approximate daily dose on oral administration is about 150 mg to about 1500 mg, advantageously in several equal part-doses.

The examples which follow illustrate the invention described above ; however, they are not intended to restrict the scope of the latter in any way. Temperatures are stated in degrees celsius.

The chemical name of the basic frameworks from which the rifamycin derivatives of the present invention are derived is as follows :

1-deoxy-15-deoxo-1,15-oxy-rifamycin SV (A) [$R_3$ and $R_3'$ denote a common bond] and 16,17,18,19-tetrahydro-11-hydroxy-11,15-dideoxo-1-deoxy-1,15-oxy-rifamycin SV (B) [$R_3$ and $R_3'$ each denote hydrogen] can be represented as follows are understood in this text to include the specific stereochemical configurations depicted below :

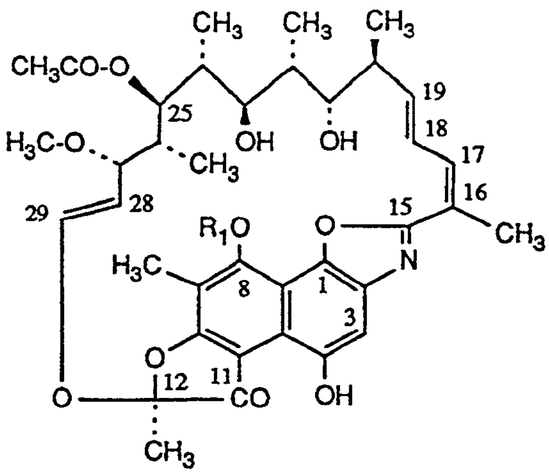

(A)

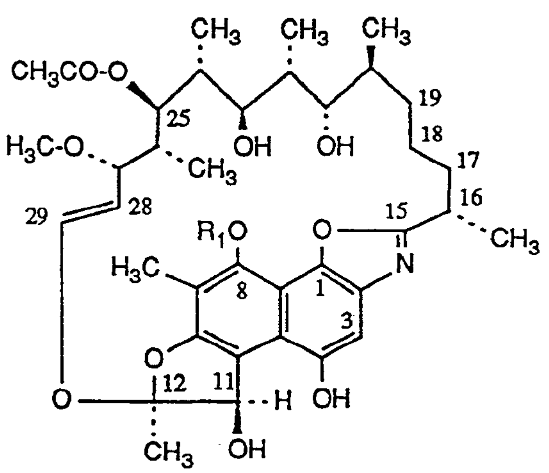

(B)

In the following Examples, melting points (m.p.) are given in degrees celsius.

Example 1 : A solution of 1 g of 8-O-pivaloyl-1-deoxy-15-deoxo-1,15-oxy-3-[4-(2,4,6-trimethylbenzyl)-piperazin-1-yl]-rifamycin SV in 20 ml of acetone is treated with 1.3 g of $K_2CO_3$ in 0.48 ml of dimethyl sulfate and heated under reflux. After three hours, the mixture is cooled, concentrated in vacuo and taken up in 40 ml of

methylene chloride and 20 ml of water. The organic phase is washed with 20 ml of brine and dried with $MgSO_4$. The crude product is purified by flash chromatography on silica gel with the eluent ethyl acetate/hexane 1 : 2. 4-O-Methyl-8-O-pivaloyl-1-deoxy-15-deoxo-1,15-oxy-3-[4-(2,4,6- trimethylbenzyl)-piperazin-1-yl]-rifamycin SV is obtained in this way as a yellow amorphous solid, melting point 157-161°.

The starting material is prepared as follows :

Step 1.1) A mixture of 5 g of 3-[4-(2,4,6-trimethylbenzyl)-piperazin-1-yl]-rifamycin SV [WO 87/02361], 50 ml of dry pyridine and 4.5 ml of pivaloyl chloride is maintained at 50° for 30 minutes. The solvent is then evaporated in vacuo. The oily residue is dissolved in ethyl acetate and washed with 2N hydrochloric acid, with buffer solution pH = 7 and with brine. After drying over sodium sulfate and evaporation, the yellow residue is crystallized from ether/hexane. Obtained in this way is 1,8-di-O-pivaloyl-3-[4-(2,4,6-trimethylbenzyl)-piperazin-1-yl)-rifamycin SV of melting point 203-204°. $C_{61}H_{83}N_3O_{16}$ ; MW : 1081 (found, MS).

Step 1.2) 30 g of 1,8-di-O-pivaloyl-3-[4-(2,4,6-trimethylbenzyl)-piperazin-1-yl]-rifamycin SV are dissolved in 1000 ml of hot 2-methoxyethanol and, under nitrogen, boiled under reflux for 5 hours. The solvent is then evaporated in vacuo, and the residue is crystallized twice from methanol. The result is 8-O-pivaloyl-1-deoxy-15-deoxo-1,15-oxy-3-[4-(2,4,6-trimethylbenzyl)-piperazin-1-yl]-rifamycin SV of the formula I in which the structural elements $-A_1-A_2-$, $-A_3-A_4-$ and $-A_5-A_6-$ each denote vinylene, X represents $>N-$ , alk denotes methylene, $R_1$ denotes pivaloyl, $R_2$ and $R_7$ each denote hydrogen, $R_3$ and $R_3'$ together represent a bond, $R_4$ denotes 2,4,6-trimethylphenyl and $R_5$ is acetyl. Melting point 160-165°. $C_{56}H_{73}N_3O_{12}$ ; M = 979, found (MS) : 979 ; $^1H$ NMR (360 MHz, $CDCl_3$, TMS) : 1.49 (s, 9H, pivaloyl on O-8).

Example 2 : A solution of 0.5 g of 16,17,18,19,-tetrahydro-8-O-pivaloyl-1-deoxy-15-deoxo-1,15-oxy-3-[4-(2,4,6-trimethylbenzyl)-piperazin-1-yl]-rifamycin SV in 15 ml of acetone is treated with 0.57 g of $K_2CO_3$ and 0.1 g of dimethyl sulfate and heated under reflux. After 18 hours, the mixture is cooled, concentrated in vacuo and taken up in 20 ml of methylene chloride and 20 ml of water. The organic phase is washed with brine and dried over $MgSO_4$. The crude product is purified by flash chromatography on silica gel, eluting with ethyl acetate-/hexane 1 : 3 via 1 : 2 to 2 : 1. The first main product, 16,17,18,19-tetrahydro-4-O-methyl-8-O-pivaloyl-1-deoxy-15-deoxo-1,15-oxy-3-[4-(2,4,6-trimethylbenzyl)-piperazin-1-yl]-rifamycin SV, isolated as a yellow amorphous solid, melting point 138 - 140°.

The starting material is prepared as follows, for example :

Step 2.1) A solution of 2 g of 8-O-pivaloyl-1-deoxy-15-deoxo-1,15-oxy-3-[4-(2,4,6-trimethylbenzyl)-piperazin-1-yl]-rifamycin SV is hydrogenated in 100 ml of ethanol in the presence of 0.2 g of palladium on charcoal (10 %) at 25° under atmospheric pressure until hydrogen uptake has ceased. The catalyst is then removed by filtration, and the solvent is evaporated in vacuo. The dark-red residue is chromatographed on 200 g of silica gel with the eluent petroleum ether/ethyl acetate (3 : 1). The eluate of the main band is collected and evaporated. The residue consists of epimerically pure 16,17,18,19-tetrahydro-8-O-pivaloyl-1-deoxy-15-deoxo-1,15-oxy-3-[4-(2,4,6-trimethylbenzyl)-piperazin-1-yl]-rifamycin SV. MW : 983 (found : MS).

Example 3 : 0.5 g of $K_2CO_3$ and 0.1 ml of dimethyl sulfate are added to a stirred solution of 0.75 g of 16,17,18,19,28,29-hexahydro-11-hydroxy-8-O-pivaloyl-1-dideoxy-11,15-dideoxo-1,15-oxy-3-[4-(2,4,6-trimet hylbenzyl)-piperazin-1-yl]-rifamycin SV in 10 ml of acetone. After stirring at 25° for 18 hours, the mixture is concentrated in vacuo and taken up in 20 ml of ethyl acetate and 10 ml of water. The organic phase is washed with brine and dried with $MgSO_4$. The crude product is purified by flash chromatography, eluting with ether/hexane 2 : 1 to 3 : 1. 16,17,18,19,28,29-Hexahydio-11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV is obtained in this way as a pale pink-coloured solid, melting point 140-145°.

The starting material is prepared as follows :

Step 3.1) 5 g of the reaction product from Step 1.2 are dissolved in 500 ml of ethanol and hydrogenated in the presence of 0.5 g of $PtO_2$ and 0.7 g of sulfuric acid at room temperature and under atmospheric pressure until 4 equivalents of hydrogen (~ 460 ml) have been taken up. The catalyst is removed by filtration, the filtrate is neutralized with aqueous sodium bicarbonate solution, saturated brine is added, and the hydrogenation product is extracted by repeated shaking with ethyl acetate. After drying and evaporation of the ethyl acetate extract, the residue is dissolved in ether. After standing for some time there is crystallization of 16,17,18,19,28,29-hexahydio-8-O-pivaloyl-11-hydroxy-1-deoxy-11,15-dideoxo-1,15-oxy-3-[4-(2,4,6-trimethyl-benzyl)-piperazin-1-yl]-rifamycin SV of the formula I in which the structural elements $-A_1-A_2-$, $-A_3-A_4-$ and $-A_5-A_6-$ each denote ethylene, X represents $>N-$ , alk denotes methylene, $R_1$ denotes pivaloyl, $R_2$, $R_3$, $R_3'$

and $R_7$ each denote hydrogen, $R_4$ denotes 2,4,6-trimethylphenyl and $R_5$ is acetyl. The pale brownish-red crystals, which form colourless plates after recrystallization from ether, melt at 225-227° (decomposition). $C_{56}H_{81}N_3O_{12}$ ; MW : 987 (found ; MS,FD).

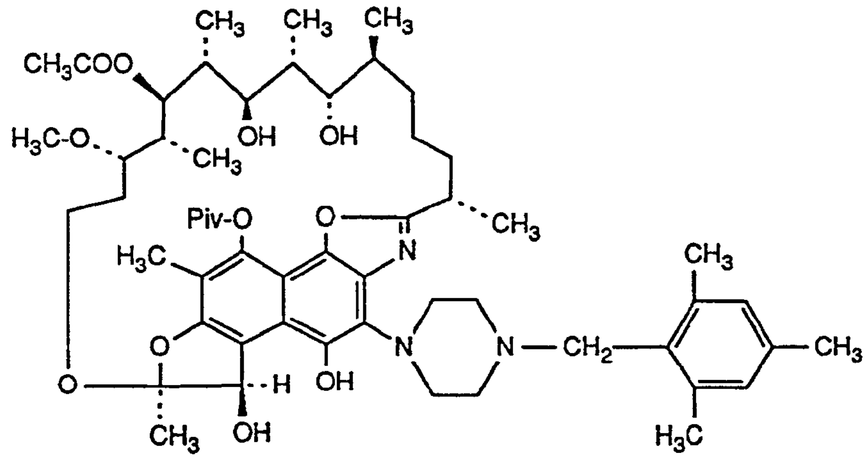

Example 4 : 0.55 g of $K_2CO_3$ and 0.2 ml of dimethyl sulfate are added to a stirred solution of 1 g of 16,17,18,19,28,29-hexahydro-4-O-acetyl-11-hydroxy-8-O-pivaloyl-1-deoxy-11,15-dideoxo-1,15-oxy-3-[4-(2,4,6-methylbenzyl)-piperazin-1-yl]-rifamycin SV in 20 ml of acetone. After stirring at 25° for 18 hours, the mixture is concentrated in vacuo and taken up in 50 ml of ethyl acetate and 20 ml of water. The organic phase is washed with brine and dried with $MgSO_4$. The crude product is purified by flash chromatography, eluting with ethyl acetate/hexane 1 : 2 to 1 : 1. 16,17,18,19,28,29-Hexahydro-11-acetyl-oxy-4-O-methyl-8-O-pivaloyl-1-deoxy-11,15-dideoxo-1,15-oxy-3-[4-(2,4,6-trimethylbenzyl-piperazine-1-yl]-rifamycin SV is obtained in this way as a white amorphous solid, melting point 125-130°.

Example 5 : A solution of 750 mg of 8-O-pivaloyl-1-deoxy-15-deoxo-1,15-oxy-3-[4-(2,4,6-trimethylbenzyl)-piperazin-1-yl]-rifamycin SV (cf. step 1.2) in 15 ml of acetone is treated with 420 mg of $K_2CO_3$ and 0.65 ml of ethyl iodide and heated under reflux. After 18 hours, the mixture is cooled, concentrated in vacuo and taken up in 40 ml of methylene chloride and 20 ml of water. The organic phase is washed with 20 ml of brine and dried with $MgSO_4$. The crude product is purified by flash chromatography, eluting with ethyl acetate/hexane 1: 3 via 1 : 2 to 1 : 1. 4-O-Ethyl-8-O-pivaloyl-1-deoxy-15-deoxo-1,15-oxy-3-[4-(2,4,6-trimethylbenzyl)-piperazin-1-yl]-rifamycin SV is obtained in this way as a yellow amorphous solid, melting point 154-157°.

Example 6 : A solution of 750 mg of 8-O-pivaloyl-1-deoxy-15-deoxo-1,15-oxy-3-[4-(2,4,6-trimethylbenzyl)-piperazin-1-yl]-rifamycin SV (cf. step 1.2) in 15 ml of acetone is treated with 420 mg of $K_2CO_3$ and 0.23 ml of benzyl bromide and heated under reflux. After 4.5 hours, the mixture is cooled, concentrated in vacuo and taken up in 40 ml of methylene chloride and 20 ml of water. The organic phase is washed with 20 ml of brine and dried with $MgSO_4$. The crude product is purified by flash chromatography, eluting with ethyl acetate/hexane 1: 3 to 1 : 2. 4-O-Benzyl-8-O-pivaloyl-11-hydroxy-1-deoxy-11,15-dideoxo-1,15-oxy-3-[4-(2,4,6-trimethylbenzyl)-piperazin-1-yl]-rifamycin SV is obtained in this way as a yellow amorphous solid, melting point 142-146°.

Example 7 : A solution of 0.5 g of 16,17,18,19,28,29-hexahydro-11-hydroxy-8-O-pivaloyl-1-deoxy-11,15-dideoxo-1,15-oxy-3-[4-(2,4,6-trimethylbenzyl)-piperazin-1-yl]-rifamycin SV (cf. step 3.1) in 15 ml of acetone is treated with 0.28 g of $K_2CO_3$ and 0.5 ml of ethyl iodide and heated under reflux. After 24 hours, the mixture is cooled, concentrated in vacuo and taken up in 40 ml of methylene chloride and 20 ml of water. The organic phase is washed with 20 ml of brine and dried with $MgSO_4$. The crude product is purified by flash chromatography, eluting with ethyl acetate/hexane 1 : 2 to 1 : 3. 16,17,18,19,28,29-Hexahydro-4-O-ethyl-11-hydroxy-8-O-pivaloyl-1-deoxy-11,15-dideoxo-1,15-oxy-3-[4-(2,4,6-trimethylbenzyl)-piperazin-1-yl]-rifamycin SV is obtained in this way as an amorphous solid, melting point 122-126°.

Example 8 : A solution of 0.5 g of 16,17,18,19,28,29-hexahydro-11-hydroxy-8-O-pivaloyl-1-deoxy-11,15-dideoxo-1,15-oxy-3-[4-(2,4,6-trimethylbenzyl)-piperazin-1-yl]-rifamycin SV (cf. step 3.1) in 15 ml of acetone is treated with 0.28 g of $K_2CO_3$ and 0.5 ml of benzyl bromide and heated under reflux. After 3 hours, the mixture is cooled, concentrated in vacuo and taken up in 40 ml of methylene chloride and 20 ml of water. The organic phase is washed with 20 ml of brine and dried with $MgSO_4$. The crude product is purified by flash chromatography, eluting with ethyl acetate/hexane 1 : 2 to 1 : 3. 16,17,18,19,28,29-Hexahydro-4-O-benzyl-11-hydroxy-8-O-pivaloyl-1-deoxy-11,15-dideoxo-1,15-oxy-3-[4-(2,4,6-trimethylbenzyl)-piperazin-1-yl]-rifamycin SV is obtained in this way as an amorphous solid, melting point 114-118°.

Example 9 : A solution of 1 g of 8-O-pivaloyl-1-deoxy-15-deoxo-1,15-oxy-3-[4-(2,4,6-trimethylbenzyl)-piperazin-1-yl]-rifamycin SV (cf. step 1.2) in 20 ml of tetrahydrofuran and 20 ml of water is treated with 38 mg of $NaBH_4$ and stirred at 25° for 10 minutes. Then 1 ml of methanol is added and, after 10 minutes, the mixture is concentrated in vacuo and the solvent including water to remove. The yellow solid residue is taken up in 20 ml of acetone and treated with 0.42 g of $K_2CO_3$ and 0.2 ml of dimethyl sulfate and stirred. After 18 hours, the mixture is concentrated in vacuo and taken up in 40 ml of ethyl acetate and 10 ml of water. The organic phase is washed with 20 ml of brine, and with $MgSO_4$. The crude product is purified with flash chromatography on silica gel, eluting with ether/hexane 1 : 1. 11-Hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV is obtained in this way as an amorphous brownish solid, melting point 155-160°.

Example 10 : In an analogous manner, for example as described in one of the preceding examples, it is possible to prepare :

11-Hydroxy-4-O-methyl-8-O-pivaloyl-1-dideoxy-11,15-deoxo-1,15-oxy-3-(4-cyclohexylmethyl-piperazin-1-yl) -rifamycin SV, m.p. 130-134°.

16,17,18,18,19,28,29-Hexahydro-11-hydroxy-4-O-methyl-8-O-pivaloyl-1-deoxy-11,15-dideoxo-1,15-oxy-3-(4 -cyclohexylmethyl-piperazin-1-yl)-rifamycin SV.

Example 11 : A solution of 1.0 g of 16,17,18,19-tetrahydro-8-O-pivaloyl-1-deoxy-15-deoxo-1,15-oxy-3-[4-(2,4,6-trimethylbenzyl)-piperazin-1-yl]-rifamycin SV (cf. step 2.1) in 20 ml of tetrahydrofuran and 0.5 ml of water is treated with 45 mg of $NaBH_4$ and stirred for 10 minutes at 25° followed by a quench with 1 ml of methanol. The mixture is concentrated in vacuo in order to remove the solvents including water. The residual yellow solid is taken up in 20 ml of acetone and treated with 690 mg of $K_2CO_3$ and 0.2 ml of dimethylsulphate and stirred. After 18 hours the mixture is concentrated in vacuo and taken up in 40 ml of ethylacetate and 10 ml of water. The organic phase is washed with 20 ml of brine and dried with $MgSO_4$. The crude product is purified with flash chromatography over silica gel eluting with ethylacetate/hexane 1 : 3. The product, 16,17,18,19-tetrahydro-11-hydroxy-4-O-methyl-8-O-pivaloyl-1-deoxy-11,15-dideoxo-1,15-oxy-3-[4-(2,4,6-trimethylbenzyl)-piperazin-1-y l]-rifamycin SV, cristallises in the form of colorless needles, m.p. 255-258°. $^1$NMR (300 MHz ; DMSO, 80°) : d 6,82 (s, 2H) ; 6,30 (d, 1H) ; 5,70 (s, 1H) ; 3,88 (s, 3H).

Example 12 : A solution of 5.0 g of 11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV (cf. example 9) in 200 ml of ethanol is hydrogenated in presence of 1 g of 10% Pd-C (Engelhard). After stirring for 18 hours the catalyst is filtered off and the solvent removed in vacuo. The crude product is cristallised from ethanol/water. Thus, 16,17,18,19-tetrahydro-11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV is isolated, m.p. 255-258°.

Example 13 : A mixture of 8-O-pivaloyl-1-deoxy-15-deoxo-1,15-oxy-3-(4-neopentyl-piperazin-1-yl)-rifamycin SV (700 mg) in tetrahydrofuran (25 ml) and water (3 ml) is treated with sodium borohydride (29 mg) under nitrogen. The color becomes pale yellow and after 5 min the reaction is quenched with acetone (5 ml). The mixture is concentrated to dryness at reduced pressure. The residue is dissolved in dimethylformamide (15 ml), degassed with nitrogen and treated with cesium carbonate (250 mg) followed by ethyl iodide (262 mg) under nitrogen. After 45 min stirring, it is quenched with ice-cold brine solution (50 ml) and extracted with ether. The ether extracts are washed with brine, dried over magnesium sulphate and concentrated to dryness at reduced pressure. The residue is flash chromatographed over silica with 2 : 1 hexane-ethyl acetate as eluent to give the pure product, 4-O-ethyl-11-hydroxy-8-O-pivaloyl-1-deoxy-11,15-dideoxo-1,15-oxy-3-(4-neopentyl-piperazin-1-yl)-rifamycin SV, m.p. 154-157°.

The starting material can be prepared, for example, in the following manner :

Bromine (0.63 ml) is added slowly to a mixture of rifamycin S (5 g), ethyl acetate (60 ml) and pyridine (1.74 ml) at -10° under nitrogen with stirring. After 2 hours at -5°, the solution is washed with 5% cold aqueous sodium thiosulphate (20 × 2 ml), then with cold water, dried over magnesium sulphate and filtered. The solution is treated at room temperature with 1-neopentyl-piperazine and triethylamine (1.2 ml). After 18.5 hours, the mixture is concentrated to dryness at reduced pressure to a dark residue, redissolved in ethyl acetate (100 ml), washed with saturated sodium bicarbonate solution, then with brine, dried over magnesium sulphate and concentrated to dryness at reduced pressure. The residue is flash chromatographed over silica with 3 % methanol in methylene chloride as eluent to purify the desired 3-(4-neopentyl)-piperazin-1-yl-rifamycin. This solid in methanol (80 ml) is treated with a solution of sodium ascorbate (3 g) in water (15 ml) and methanol (25 ml) dropwise over 40 minutes. It is concentrated to ca. 30 ml at reduced pressure, extracted with methylene chloride and the organic layer is dried over magnesium sulphate and concentrated to dryness at reduced pressure. The residual orange solid is dissolved in ethyl acetate, cooled to -20° and treated with 4-dimethylaminopyridine (13 mg) and pivaloyl chloride (0.5 ml). Triethylamine (0.7 ml) is added slowly at -20° and stirred 23 hours at -10°. It is treated with ethyl acetate (15 ml), pivaloyl chloride (0.25 ml) and triethylamine (0.35 ml) for 1 hour to remove

all starting material. The mixture is quenched in water (30 ml) and stirred 10 minutes at ice temperature. The organic layer is washed with saturated sodium bicarbonate (30 × 2 ml), water (30 ml) and then brine and dried over magnesium sulphate to afford a solid. This is dissolved in 2-methoxyethanol (70 ml) and heated at reflux under nitrogen for 70 minutes. The mixture is concentrated to dryness at reduced pressure to afford the starting material m.p. 130-140° dec..

Example 14 : When an equivalent amount of dimethylsulfate is used in place of ethyl iodide in Example 13, the product obtained is 11-hydroxy-4-O-methyl-8-O-pivaloyl-1-deoxy-11,15-dideoxo-1,15-oxy-3-(4-neopentylpiperazin-1-yl)-rifamycin SV.

Example 15 : A mixture of 8-O-pivaloyl-1-deoxy-15-deoxo-1,15-oxy-3-(4-cyclohexylmethylpiperidin-1-yl)-rifamycin SV (500 mg) and tetrahydrofuran (10 ml) cooled to -10°C is treated with sodium borohydride (41 mg) in water (0.5 ml) under nitrogen. After 30 minutes at -10° to 0°C, the solvent is evaporated under vacuum and the residue is dissolved in acetone (15 ml) and treated with potassium carbonate (345 mg) and dimethyl sulphate (0.105 ml). After overnight stirring under nitrogen, the solvent is removed at reduced pressure and the residue partitioned between ethyl acetate (100 ml) and water (25 ml). The organic layer is washed with brine, dried over magnesium sulphate and concentrated to dryness at reduced pressure. The residue is flash chromatographed over silica gel with 1 : 2 ethyl acetate-hexane as eluent to afford 11-hydroxy-4-O-methyl-8-O-pivaloyl-1-deoxy-11,15-dideoxo-1,15-oxy-3-(4-cyclohexylmethylpiperidin-1-yl)-rifamycin SV, m.p. 112-115°, $[\alpha]_D^{25}$ = +435.6, c=3.2, $CH_2Cl_2$.

The starting material can be prepared, for example, in the following manner :

To a solution of 3-(4-cyclohexylmethylpiperidin-1-yl)-rifamycin (2.87 g, prepared as described by Traxler et al., J. Med. Chem. 1990, 552-560), methylene chloride (80 ml), triethylamine (1.25 ml) and 4-dimethylaminopyridine (20 mg) is added, with stirring under nitrogen at -5° to 0°C, pivaloyl chloride (1.08 ml). After 1 hour, the mixture is washed with water, then with brine, dried over magnesium sulphate and concentrated to dryness at reduced pressure. The residue is flash chromatographed over silica gel with ethyl acetate-hexane as eluent (1 : 2 followed by 3 : 5). Evaporation of the fractions containing only the desired material gives 1,8-di-O-pivaloyl-3-(4-cyclohexylmethylpiperidin-1-yl)-rifamycin, m.p. 165-170°. This intermediate is dissolved in dl-1-methoxy-2-propanol (25 ml) and heated under nitrogen at 135°C for 5.5 hours. The solvent is removed at reduced pressure and the residue chromatographed on silica gel with ethyl acetate-hexane as eluent (1 : 3 followed by 1 : 2). The desired fractions yield pure 8-O-pivaloyl-1-deoxy-15-deoxo-1,15-oxy-3-(4-cyclohexylmethylpiperidin-1-yl)-rifamycin SV as a pink solid, m.p. 122-125°.

Example 16 : A solution of 4-O-methyl-8-O-pivaloyl-1-deoxy-15-deoxo-1,15-oxy-3-[4-(2,4,6-trimethylbenzyl)-piperazin-1-yl]-rifamycin SV (cf. example 1) (150 mg) in tetrahydrofuran (2 ml) is treated with sodium borohydride (12 mg) and water (0.5 ml) under nitrogen. After 10 minutes at room temperature, more borohydride (24 mg) is added and stirring is continued for 30 minutes. The solvents are removed under vacuum and the residue partitioned between ethyl acetate and water. The organic layer is dried over magnesium sulphate and concentrated to dryness at reduced pressure to afford pure 11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV, m.p. 175-180°, identical to the product of Example 9.

The starting material can be prepared, for example, in the following manner :

A mixture of 8-O-pivaloyl-1-deoxy-15-deoxo-1,15-oxy-3-[4-(2,4,6-methylbenzyl)-piperazin-1-yl]-rifamycin SV (400 mg), 2,2-dimethoxypropane (10 ml), 10-camphorsulfonic acid (110 mg) and acetone (10 ml) is stirred overnight under nitrogen. The mixture is concentrated to dryness at reduced pressure and partitioned between ethyl acetate and saturated sodium bicarbonate solution. The organic layer is washed with brine, dried over magnesium sulphate and concentrated to dryness at reduced pressure to afford amorphous solid 21,23-di-O-isopropylidene-8-O-pivaloyl-1-deoxy-15-deoxo-1,15-oxy-3-[4-(2,4,6-trimethylbenzyl)-piperazin-1-yl]-rifamycin (350 mg). This is dissolved in dimethylformamide (3 ml), cooled to 0° and treated with sodium hydride (20 mg, 60% in oil) under nitrogen. After 5 minutes at 0-5°, the mixture is treated with dimethyl sulfate (0.04 ml) and stirred 1 hour at ambient temperature. The mixture is concentrated to dryness at reduced pressure and partitioned between ether and saturated sodium bicarbonate. The organic layer is washed with brine, dried over sodium sulphate and concentrated to dryness at reduced pressure. The residue is purified through a column of silica gel with 1 : 3 ethyl acetate-hexane as eluent to afford pure 21,23-di-O-isopropylidene-4-O-methyl-8-O-pivaloyl-1-deoxy-15-deoxo-1,15-oxy-3-[4-(2,4,6-trimethylbenzyl)-piperazin-1-yl]-rifamycin SV as an amorphous solid. The product (200 mg) in tetrahydrofuran (3 ml) is treated with 20% aqueous sulphuric acid (3 ml) at room temperature for 1 hour under nitrogen. The mixture is diluted with brine and extracted with ether. The ether extract is washed with saturated sodium bicarbonate and dried over sodium sulphate to afford the desired starting material as a yellow amorphous solid.

Example 17 : By the same sequence of reactions as described in Example 16, 8-O-pivaloyl-1-deoxy-15-deoxo-1,15-oxy-3-(4-cyclohexylmethylpiperazin-1-yl)-rifamycin SV can be converted to 4-O-methyl-8-O-pivaloyl-1-deoxy-15-deoxo-1,15-oxy-3-(4-cyclohexylmethylpiperazin-1-yl)-rifamycin SV, m.p. 115-120°, and then similarly reduced with sodium borohydride to give 11-hydroxy-4-O-methyl-8-O-pivaloyl-1-deoxy-11,15-dideoxo-1,15-oxy-3-(4-cyclohexylmethylpiperazin-1-yl)-rifamycin SV, m.p. 130-134°.

The above reduction is carried out as follows :

A solution of 390 mg of 4-O-methyl-8-O-pivaloyl-1-deoxy-15-deoxo-1,15-oxy-3-(4-cyclohexylmethyl-piperazin-1-yl)-rifamycin SV in 3 ml tetrahydrofuran is treated with a solution of 30 mg of sodium borohydride in 0.5 ml of water at room temperature. According to thin layer chromatography in ethyl acetate-hexane (1 : 1) the reaction is completed after 1 hour. The reaction mixture is quenched with acetone and concentrated under vacuum. The crude product is purified by flash chromatography on silica gel and eluted with ethyl acetate-hexane (1 : 4-1 : 2). 11-hydroxy-4-O-methyl-8-O-pivaloyl-1-deoxy-11,15-dideoxo-1,15-oxy-3-(4-cyclohexylmethyl-piperazin-1-yl)-rifamycin SV is obtained as an amorphous solid, m.p. 130-134° (decomposition), $[\alpha]_D^{25}$ = +487.89.

Example 18 : The product from Example 9 (320 mg) in methanol (18 ml) is reacted with hydrazine hydrate (3 ml) under argon for 5 days at room temperature. It is treated cautiously with acetone, concentrated to dryness at reduced pressure and retreated with acetone in the same manner several times. The residue is taken up in ether and washed with dilute hydrochloric acid (pH 2-3). The ether solution is washed with water, dried over magnesium sulphate, concentrated to dryness at reduced pressure and flash chromatographed over silica gel with 1 : 3 ethyl acetate-hexane as eluent. The first product eluted is pure 25-deacetyl-11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV, m.p. 156-160°.

Example 19 : A solution of 8-O-pivaloyl-1-deoxy-15-deoxo-1,15-oxy-3-[4-(2,4,6-trimethylbenzyl)-piperazin-1-yl] -rifamycin SV (10 g, cf. step 1.2) in tetrahydrofuran (150 ml) under nitrogen is treated with sodium borohydride (770 mg) in water (10 ml). After stirring at 0-5° for 15 minutes, the reaction mixture is quenched with acetone (10 ml) and the solvent removed under water vacuum and the residue further dried under high vacuum. The residue is taken up in acetone (200 ml) and stirred under nitrogen with potassium carbonate (7 g) and dimethyl sulphate (1.5 ml) for 18 hours. The mixture is filtered and the filtrate concentrated to dryness at reduced pressure to give a dark residual solid. This is taken up in ethyl acetate (400 ml) washed with water (3 × 30 ml) then with brine and dried over magnesium sulphate. Evaporation of the solvent gives a yellow amorphous solid. It is dissolved in ethanol (60 ml) and treated with one equivalent of oxalic acid. The resulting solution is poured into water (600 ml) and the precipitate is collected and partitioned between ethyl acetate and saturated aqueous sodium bicarbonate. The organic layer is concentrated to a solid which is further purified by chromatography on silica gel with ethyl acetate-hexane as eluent (1 : 3 initially, then 1 : 2). Fractions containing only the desired product are concentrated to dryness to afford the 11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV, m.p. 155-160° (dec), $[\alpha]_D^{25}$ = +495.8°, c=0.9, $CH_2Cl_2$, as a white amorphous solid.

Example 20 : Capsules containing 250 mg of active compound, for example the compound of the formula I in which the structural elements $-A_1-A_2-$, $-A_3-A_4-$ and $-A_5-A_6-$ each denote vinylene, X represents 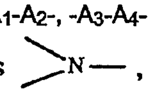 , alk denotes methylene, $R_1$ denotes pivaloyl, $R_2$ denotes methyl, $R_3$, $R_3'$ and $R_7$ each denote hydrogen, $R_4$ denotes 2,4,6-trimethylphenyl and $R_5$ is acetyl can be prepared as follows :

| Composition (for 1000 capsules): | |
|---|---|
| Active Compound | 250.0 g |
| Maize starch | 50.0 g |
| Polyvinylpyrrolidone | 15.0 g |
| Magnesium stearate | 5.0 g |
| Ethanol | q.s. |

The active compound and the maize starch are mixed and moistened with a solution of polyvinylpyrrolidone in 50 g of ethanol. The moist composition is forced through a screen with a mesh width of 3 mm and dried at 45°. The dry granules are screened through a screen with a mesh width of 1 mm and mixed with 5 g of magnesium stearate. The mixture is dispensed in 0.320 g portions into size 0 two-piece capsules.

In an analogous manner pharmaceutical compositions containing as active component one of the other compounds of the formula I or a pharmaceutically acceptable salt therof, prepared as in the preceding examples, are obtained.

## Claims

1. Rifamycin SV derivatives of the formula

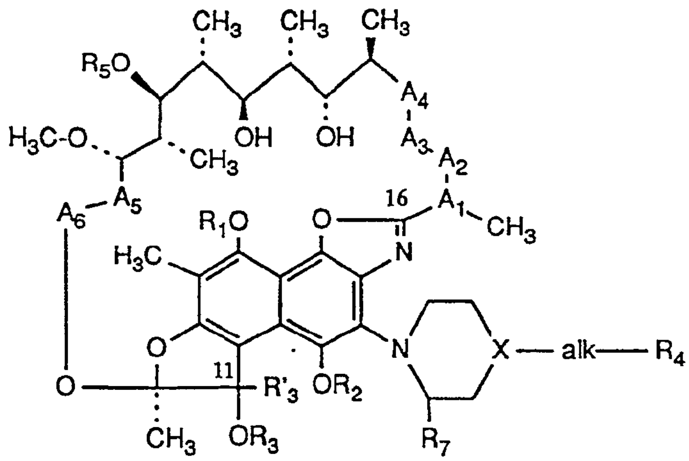

(I)

and the salts thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene; X represents $>C(R_6)-$ or $>N-$ and $R_6$ denotes hydrogen or alkyl ; alk denotes an aliphatic hydrocarbon radical ; $R_1$ denotes hydrogen or acyl ; $R_2$ denotes alkyl which is optionally substituted by an aromatic radical ; $R_3$ and $R_3'$ represent a common bond, or $R_3$ denotes hydrogen or acyl, and $R_3'$ is hydrogen ; $R_4$ denotes hydrogen, cycloalkyl or aryl ; $R_5$ denotes hydrogen or acetyl ; and $R_7$ denotes hydrogen or alkyl.

2. Rifamycin SV derivatives according to claim 1 of the formula I, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene ; X represents $>C(R_6)-$ or $>N-$ and $R_6$ denotes hydrogen or alkyl; alk denotes an aliphatic hydrocarbon radical ; $R_1$ denotes hydrogen or acyl ; $R_2$ denotes alkyl which is optionally substituted by an aromatic radical ; $R_3$ and $R_3'$ represent a common bond, or $R_3$ denotes hydrogen or acyl, and $R_3'$ is hydrogen ; $R_4$ denotes hydrogen, cycloalkyl or aryl ; $R_5$ denotes hydrogen or acetyl ; and $R_7$ denotes hydrogen or alkyl ; and wherein if $-A_1-A_2-$ denotes ethylene, the methyl group bonded to $A_1$ is in the α-position, and if $R_3'$ denotes hydrogen, $R_3'$ is in the α-position, and the salts thereof.

3. Compounds according to claim 2 of the formula I and salts thereof, in which $-A_1-A_2-$, $-A_3-A_4-$, $-A_5-A_6-$ and $R_5$ have the indicated meanings; X represents $>C(R_6)-$ or $>N-$, and $R_6$ denotes hydrogen or $C_1$-$C_7$alkyl; alk denotes $C_1$-$C_7$alkylene, $C_3$-$C_7$alkenylene or $C_3$-$C_7$alkynylene ; $R_1$ denote $C_2$-$C_8$alkanoyl, halogen-$C_2$-$C_8$alkanoyl, phenyl- or naphthyl-$C_2$-$C_8$alkanoyl, benzoyl, naphthoyl, 5- or 6-membered monocyclic monoaza-, monooxa- or mono-

thiaaroyl, $C_1$-$C_7$alkoxycarbonyl, phenyl- or naphthyl-$C_1$-$C_7$-alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl ; $R_2$ denotes $C_1$-$C_7$alkyl, phenyl- or naphthyl-$C_1$-$C_7$alkyl ; $R_3$ and $R_3'$ represent a common bond, or $R_3$ denotes hydrogen or $C_2$-$C_8$alkanoyl, halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$-alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkylsulfonyl or benzene- or naphthylsulfonyl, and $R_3'$ is hydrogen ; $R_4$ denotes hydrogen, $C_3$-$C_7$cycloalkyl, phenyl, biphenylyl or naphthyl ; $R_7$ denotes hydrogen or $C_1$-$C_7$alkyl ; where the aromatic radicals in each case are unsubstituted or substituted one or more times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro.

4. Compounds according to claim 2 of the formula I and salts thereof, in which $-A_1$-$A_2$-, $-A_3$-$A_4$-, $-A_5$-$A_6$- and $R_5$ have the indicated meanings; X represents $>C(R_6)-$ or $>N-$, and $R_6$ is hydrogen; alk denotes $C_1$-$C_7$alkylene, $C_3$-$C_7$alkenylene or $C_3$-$C_7$alkynylene ; $R_1$ and $R_3$, independently of one another, denote $C_2$-$C_8$alkanoyl, halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene-or naphthylsulfonyl, and $R_1$ additionally and $R_3'$ and $R_7$ denote hydrogen ; $R_2$ denotes $C_1$-$C_7$alkyl, phenyl- or naphthyl-$C_1$-$C_7$alkyl ; $R_4$ denotes hydrogen, $C_3$-$C_7$cycloalkyl, phenyl, biphenylyl or naphthyl ; where the aromatic radicals are in each case unsubstituted or substituted one or more times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro.

5. Compounds according to claim 2 of the formula I and salts thereof, in which $-A_1$-$A_2$-, $-A_3$-$A_4$-, $-A_5$-$A_6$- and $R_5$ have the indicated meanings; X represents $>C(R_6)-$ or $>N-$, and $R_6$ is hydrogen; alk denotes $C_1$-$C_7$alkylene, $C_3$-$C_7$alkenylene or $C_3$-$C_7$alkynylene, in particular $C_1$-$C_4$alkylene, $C_3$-$C_4$alkenylene or $C_3$-$C_4$alkynylene, where the multiple bonds are located in a position higher than $\alpha$ to the piperazine nitrogen ; $R_1$ denotes $C_2$-$C_8$alkanoyl, phenyl-$C_2$-$C_8$alkanoyl, benzoyl, $C_1$-$C_7$alkoxy-carbonyl, phenyl-$C_1$-$C_4$alkoxycarbonyl, $C_1$-$C_4$alkanesulfonyl or benzenesulfonyl ; $R_2$ denotes $C_1$-$C_7$alkyl or phenyl-$C_1$-$C_7$alkyl ; $R_3$ and $R_3'$ together represent a bond or $R_3$ denotes hydrogen, $C_2$-$C_8$alkanoyl, phenyl-$C_2$-$C_8$alkanoyl, benzoyl, $C_1$-$C_7$alkoxy-carbonyl, phenyl-$C_1$-$C_4$alkoxycarbonyl, $C_1$-$C_4$alkanesulfonyl or benzenesulfonyl, and $R_3'$ is hydrogen ; $R_4$ denotes hydrogen, $C_3$-$C_7$cycloalkyl, phenyl, biphenylyl or naphthyl ; $R_7$ denotes hydrogen or $C_1$-$C_7$alkyl ; where the aromatic radicals in each case are unsubstituted or substituted up to three times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro.

6. Compounds according to claim 2 of the formula I and salts thereof, in which $-A_1$-$A_2$-, $-A_3$-$A_4$-, $-A_5$-$A_6$- have the indicated meanings; X represents $>C(R_6)-$ or $>N-$, and $R_6$ is hydrogen ; alk denotes $C_1$-$C_7$alkylene ; $R_1$ denotes $C_2$-$C_8$alkanoyl ; $R_2$ denotes $C_1$-$C_4$alkyl or phenyl-$C_1$-$C_4$alkyl, $R_3$ and $R_3'$ together represent a bond, or $R_3$ denotes hydrogen, $C_2$-$C_8$alkanoyl, benzoyl, $C_1$-$C_4$alkanesulfonyl or benzenesulfonyl, and $R_3'$ is hydrogen ; $R_4$ denotes hydrogen, $C_3$-$C_7$cycloalkyl, phenyl,

biphenylyl or naphthyl, $R_5$ is acetyl ; $R_7$ is hydrogen or $C_1$-$C_4$alkyl ; where the aromatic radicals in each case are unsubstituted or substituted up to three times by substituents selected from the group consisting of halogen, $C_1$-$C_4$alkyl, $C_1$-$C_4$alkoxy, hydroxyl, $C_2$-$C_6$alkanoyloxy, trifluoromethyl and nitro.

7. Compounds according to claim 2 of the formula I and salts thereof, in which $-A_1-A_2-$, $-A_3-A_4-$ and $-A_5-A_6-$ have the indicated meanings; X represents $>C(R_6)-$ or $>N-$, and $R_6$ is hydrogen; alk denotes $C_1$-$C_5$alkylene; $R_1$ denotes branched $C_3$-$C_6$alkanoyl ; $R_2$ denotes $C_1$-$C_4$alkyl or phenyl-$C_1$-$C_4$alkyl ; $R_3$ denotes hydrogen, $C_2$-$C_8$alkanoyl, benzoyl or $C_1$-$C_4$alkanesulfonyl such as methanesulfonyl, and $R_3'$ is hydrogen ; $R_4$ denotes hydrogen, $C_3$-$C_7$cycloalkyl or phenyl which is unsubstituted or substituted by up to three $C_1$-$C_4$alkyl substituents ; $R_5$ is acetyl; $R_7$ denotes hydrogen or $C_1$-$C_4$alkyl.

8. Compounds according to claim 2 of the formula I and salts thereof, in which the structural elements $-A_1-A_2-$, $-A_3-A_4-$ and $-A_5-A_6-$ each have the indicated meanings ; X represents $>N-$ ; $R_1$ denotes branched $C_3$-$C_6$alkanoyl; $R_2$ denotes $C_1$-$C_4$alkyl; $R_3$ denotes hydrogen or $C_2$-$C_5$alkanoyl ; $R_3'$ is hydrogen ; $R_5$ is acetyl ; alk-$R_4$ denotes $C_3$-$C_5$alkyl, $C_3$-$C_6$cycloalkyl-methyl or 2,4,6-tri-$C_1$-$C_4$-alkyl-benzyl ; $R_7$ is hydrogen.

9. A compound according to claim 2 of the formula I and pharmaceutically acceptable salts thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene; X represents $>CH-$ or $>N-$ ; alk denotes methylene or 2,2-dimethyl-1,3-propylene ; $R_1$ denotes pivaloyl ; $R_2$ denotes methyl, ethyl or benzyl ; $R_3$ and $R_3'$ represent a common bond, or $R_3$ denotes hydrogen or acetyl, and $R_3'$ is hydrogen ; $R_4$ denotes hydrogen, cyclohexyl or 2,4,6-trimethyl-phenyl ; $R_5$ denotes hydrogen or acetyl ; $R_7$ denotes hydrogen.

10. Compounds according to claim 2 of the formula I and salts thereof, in which the structural elements $-A_1-A_2-$, $-A_3-A_4-$ and $-A_5-A_6-$ each denote ethylene or vinylene or the elements $-A_1-A_2-$ and $-A_3-A_4$ each denote ethylene and $-A_5-A_6-$ denotes vinylene ; X represents $>N-$ ; $R_1$ denotes pivaloyl; $R_2$ denotes methyl or ethyl; $R_3$ and $R_3$' each denote hydrogen ; $R_5$ denotes acetyl ; $R_7$ is hydrogen ; and alk-$R_4$ denotes 2,4,6-trimethylbenzyl.

11. A compound according to claim 2 of the formula I selected from the group consisting of
4-O-methyl-8-O-pivaloyl-1-deoxy-15-deoxo-1, 15-oxy-3-[4-(2,4,6-trimethylbenzyl)-piperazin-1-yl]-rifamycin SV ;
16,17,18,19-tetrahydro-4-O-methyl-8-O-pivaloyl-1-deoxy-15-deoxo-1,15-oxy-3-[4-(2,4,6-trimethylbenzyl )-piperazin-1-yl]-rifamycin SV ;
16,17,18,19,28,29-hexahydro-11-hydroxy-4-O-methyl-8-O-pivaloyl-1-deoxy-11,15-dideoxo-1,15-oxy-3-[ 4-(2,4,6-trimethylbenzyl)-piperazin-1-yl]-rifamycin SV ;
16,17,18,19,28,29-hexahydro-11-acetyloxy-4-O-methyl-8-O-pivaloyl-1-deoxy-11,15-dideoxo- 1, 15-oxy-3-[4-(2,4,6-trimethylbenzyl)-piperazin-1-yl]-rifamycin SV ;
4-O-ethyl-8-O-pivaloyl-1-deoxy-15-deoxo-1,15-oxy-3-[4-(2,4,6-trimethylbenzyl)-piperazin-1-yl]-rifamycin SV ;
4-O-benzyl-11-hydroxy-8-O-pivaloyl-1-deoxy-11,15-dideoxo-1,15-oxy-3-[4-(2,4,6-trimethylbenzyl)-piper azin-1-yl]-rifamycin SV ;
16,17,18, 19,28,29-hexahydro-4-O-ethyl-11-hydroxy-8-O-pivaloyl-1-deoxy-11,15-dideoxo 1,15-oxy-3-[4-(2,4,6-trimethylbenzyl)-piperazin-1-yl]-rifamycin SV ;
16,17,18,19,28,29-hexahydro-4-O-benzyl-11-hydroxy-8-O-pivaloyl-1-deoxy-11,15-deoxo-1,15-oxy-3-[4-(2,4,6-trimethylbenzyl)-piperazin-1-yl]-rifamycin SV ;

11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV ;
11-hydroxy-4-O-methyl-8-O-pivaloyl-1-deoxy-11,15-deoxo-1,15-oxy-3-(4-cyclohexylmethyl-piperazin-1-yl)-rifamycin SV ;
16,17,18,18,19,28,29-hexahydro-11-hydroxy-4-O-methyl-8-O-pivaloyl-1-deoxy-11,15-dideoxo-1,15-oxy-3-(4-cyclohexylmethyl-piperazin-1-yl)-rifamycin SV ;
16,17,18,19-tetrahydro-11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV ;
4-O-ethyl-11-hydroxy-8-O-pivaloyl-1-deoxy-11,15-dideoxo-1,15-oxy-3-(4-neopentyl-piperazin-1-yl)-rifamycin SV ;
11-hydroxy-4-O-methyl-8-O-pivaloyl-1-deoxy-11,15-dideoxo-1,15-oxy-3-(4-neopentyl-piperazin-1-yl)-rifamycin SV ;
11-hydroxy-4-O-methyl-8-O-pivaloyl-1-deoxy-11,15-dideoxo-1,15-oxy-3-(4-cyclohexylmethylpiperidin-1-yl)-rifamycin SV ;
4-O-methyl-8-O-pivaloyl-1-deoxy-15-deoxo-1,15-oxy-3-[4-(2,4,6-trimethylbenzyl)-piperazin-1-yl]-rifamycin SV ;
and the pharmaceutically acceptable salts thereof.

12. Pharmaceutical products containing a compound of the formula I according to any one of claims 1 to 11 or a pharmaceutically utilizable salt thereof together with suitable auxiliaries and additives.

13. Method for the treatment of hypolipidaemia, characterized in that a therapeutically effective amount of a compound of the formula I according to any one of claims 1 to 11 or a pharmaceutically utilizable salt thereof is administered.

14. Use of a compound of the formula I according to any one of claims 1 to 11 or of a pharmaceutically utilizable salt thereof for the manufacture of pharmaceutical products for use for the treatment of hypolipidaemia.

15. Process for the preparation of compounds according to claim 1 of the formula I and salts thereof, characterized in that

a) for the preparation of compounds of the formula I and salts thereof, in which X represents 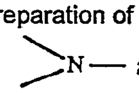 and the other symbols have the abovementioned meanings, a rifamycin SV derivative of the formula

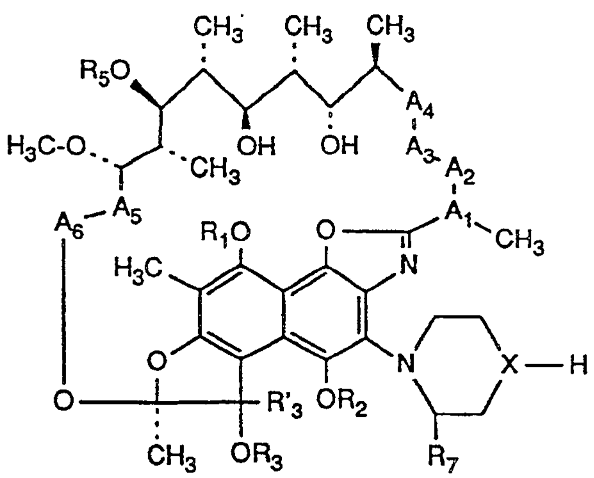

(IIa)

or a salt thereof, in which X represents 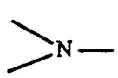, is reacted with a compound of the formula

Z-alk-$R_4$ [IIB]

in which Z denotes reactive esterified hydroxyl, or

b) a rifamycin SV derivative of the formula

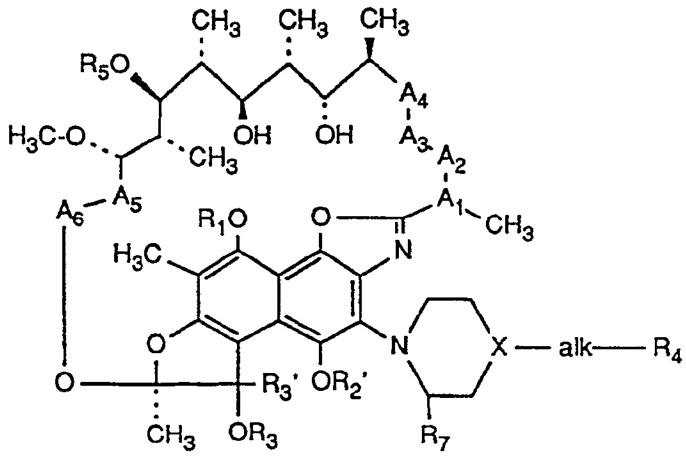

(III)

in which $R_2'$ is hydrogen or, if $R_3$ is hydrogen, $R_2'$ may also represent acyl and the other symbols have the abovementioned meanings, is alkylated and/or a rifamycin SV derivative of the formula III in which at least one of the radicals $R_1$ and $R_3$ denotes hydrogen, $R_2'$ has the abovementioned meaning of $R_2$, and the other symbols have the abovementioned meanings, is acylated, or

c) a rifamycin SV derivative of the formula

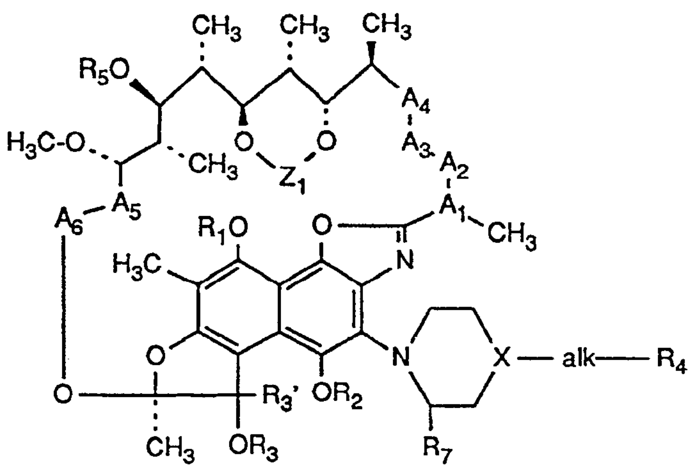

(IV)

in which $Z_1$ denotes alkylidene or cycloalkylidene and the other symbols have the abovementioned meanings, is hydrolysed, or

d) for the manufacture of compounds of the formula I wherein $R_1$ represents pivaloyl, a rifamycin SV derivative of the formula V,

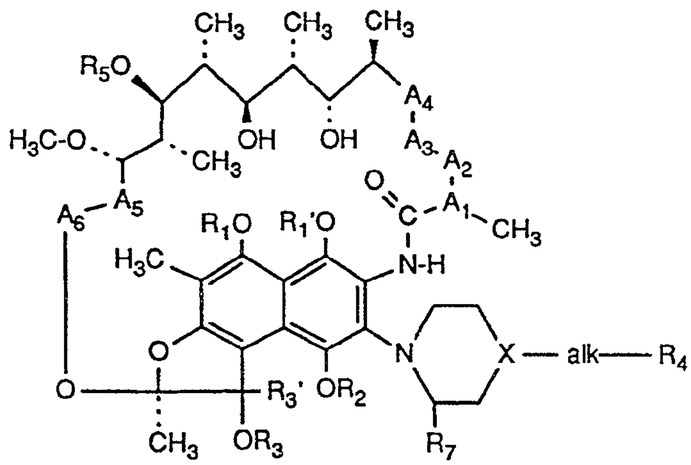

(V)

wherein $R_1$ represents pivaloyl and $R_1'$ represents pivaloyl or hydrogen and the remaining symbols have the meanings given above, is heated until cyclisation has occurred, and, if desired, a compound of the formula I obtainable according to the process or by other means, or a salt thereof, is converted into another compound according to the invention, or a salt thereof ; a free compound of the formula I obtainable according to the process is converted into a salt ; or a salt obtainable according to the process is converted into the free compound of the formula I or into another salt ; and, if desired, a mixture of isomers obtainable according to the process is fractionated.

**Claims for the following Contracting States : GR and ES**

1. Process for the manufacture of a rifamycin SV derivative of the formula

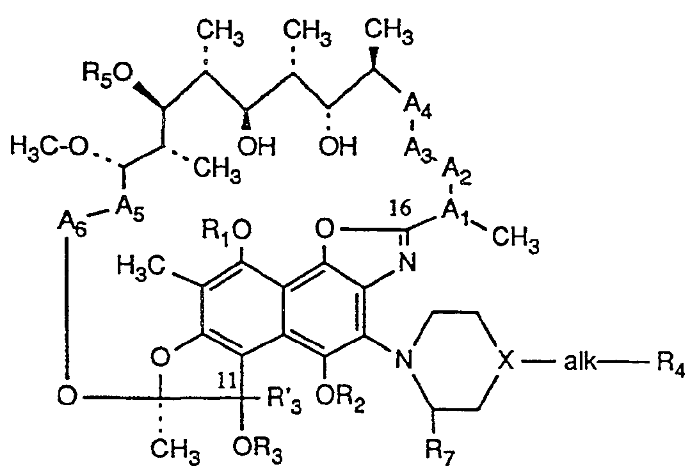

(I)

or a salt thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene; X represents $>C(R_6)-$ or $>N-$ and $R_6$ denotes hydrogen or alkyl ; alk denotes an aliphatic hydrocarbon radical ; $R_1$ denotes hydrogen or acyl ; $R_2$ denotes alkyl which is optionally substituted by an aromatic radical ; $R_3$ and $R_3'$ represent a common bond, or $R_3$ denotes hydrogen or acyl, and $R_3'$ is hydrogen ; $R_4$ denotes hydrogen, cycloalkyl or aryl ; $R_5$ denotes hydrogen or acetyl ; and $R_7$ denotes hydrogen or alkyl, characterized in that

a) for the preparation of compounds of the formula I and salts thereof, in which X represents $>N-$ and the other symbols have the abovementioned meanings, a rifamycin SV derivative of the formula

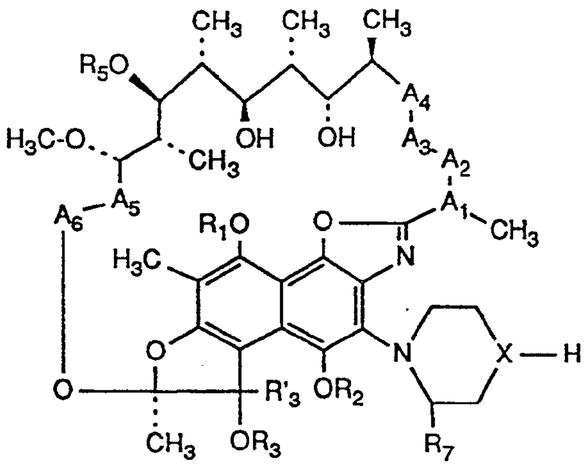

(IIa)

or a salt thereof, in which X represents $>N-$ , is reacted with a compound of the formula

Z-alk-$R_4$ [IIB]

in which Z denotes reactive esterified hydroxyl, or

b) a rifamycin SV derivative of the formula

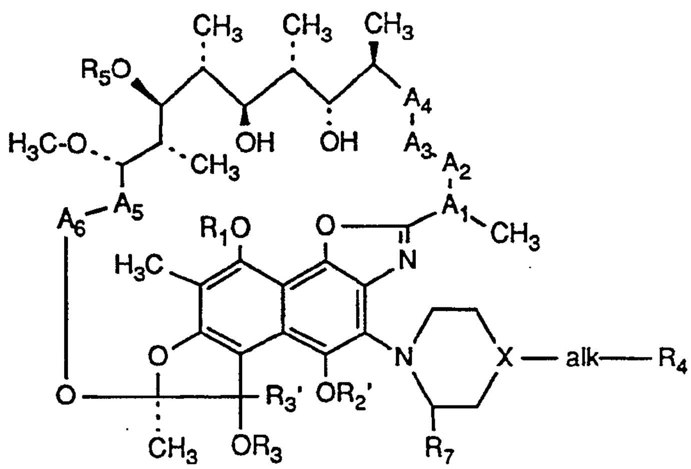

(III)

in which $R_2'$ is hydrogen or, if $R_3$ is hydrogen, $R_2'$ may also represent acyl and the other symbols have the abovementioned meanings, is alkylated and/or a rifamycin SV derivative of the formula III in which at least one of the radicals $R_1$ and $R_3$ denotes hydrogen, $R_2'$ has the abovementioned meaning of $R_2$, and the other symbols have the abovementioned meanings, is acylated, or

c) a rifamycin SV derivative of the formula

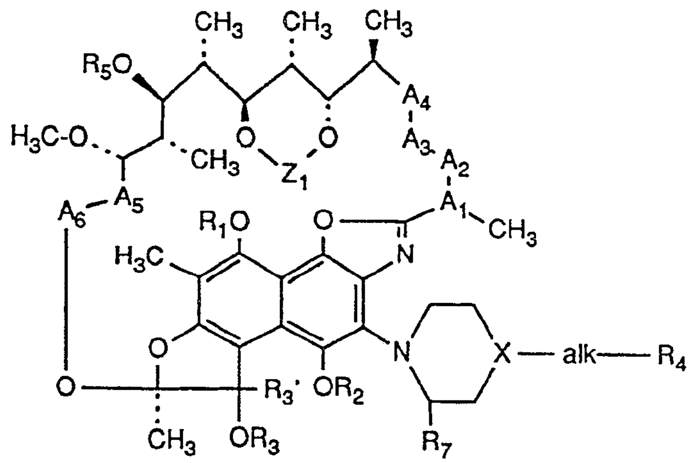

(IV)

in which $Z_1$ denotes alkylidene or cycloalkylidene and the other symbols have the abovementioned meanings, is hydrolysed, or

d) for the manufacture of compounds of the formula I wherein $R_1$ represents pivaloyl, a rifamycin SV derivative of the formula V,

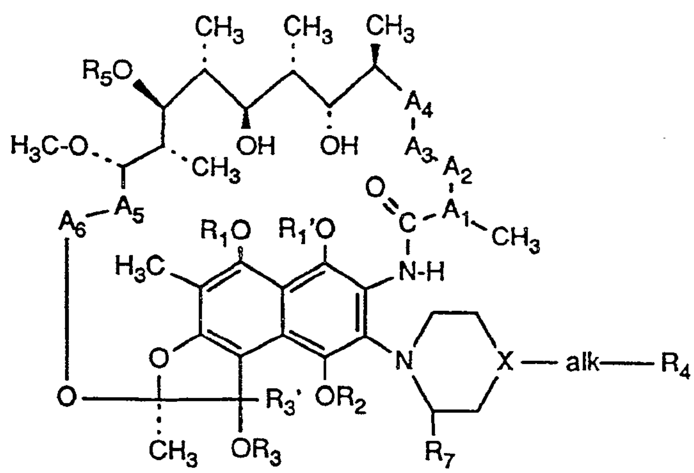

(V)

wherein $R_1$ represents pivaloyl and $R_1'$ represents pivaloyl or hydrogen and the remaining symbols have the meanings given above, is heated until cyclisation has occurred, and, if desired, a compound of the formula I obtainable according to the process or by other means, or a salt thereof, is converted into another compound according to the invention, or a salt thereof ; a free compound of the formula I obtainable according to the process is converted into a salt ; or a salt obtainable according to the process is converted into the free compound of the formula I or into another salt ; and, if desired, a mixture of isomers obtainable according to the process is fractionated.

2. Process according to claim 1 for the manufacture of a rifamycin SV derivative of the formula I, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and

$-A_5-A_6-$ denotes vinylene; X represents $>C(R_6)-$ or $>N-$ and $R_6$ denotes

hydrogen or alkyl ; alk denotes an aliphatic hydrocarbon radical ; $R_1$ denotes hydrogen or acyl ; $R_2$ denotes alkyl which is optionally substituted by an aromatic radical ; $R_3$ and $R_3'$ represent a common bond, or $R_3$ denotes hydrogen or acyl, and $R_3'$ is hydrogen ; $R_4$ denotes hydrogen, cycloalkyl or aryl ; $R_5$ denotes hydrogen or acetyl ; and $R_7$ denotes hydrogen or alkyl ; and wherein if $-A_1-A_2-$ denotes ethylene, the methyl group bonded to $A_1$ is in the α-position, and if $R_3'$ denotes hydrogen, $R_3'$ is in the α-position, or a salt thereof, characterized in that starting materials having the same configuration at the asymmetric carbon atoms as the desired end product of the formula I are used, or in that a rifamycin SV derivative of formula I, wherein

$-A_1-A_2-$ denotes vinylene and the remaining symbols have the abovementioned meanings, is reduced stereoselectively, or in that a rifamycin SV derivative of the formula I, wherein $R_3$ and $R_3'$ represent a common bond and the remaining symbols have the abovementioned meanings, is reduced stereoselectively.

3. Process according to claim 2 for the manufacture of a rifamycin SV derivative of the formula I or a salt thereof, in which $-A_1-A_2-$, $-A_3-A_4-$, $-A_5-A_6-$ and $R_5$ have the indicated meanings; X represents $>C(R_6)-$ or $>N-$, and $R_6$ denotes hydrogen or $C_1$-$C_7$alkyl ; alk denotes $C_1$-$C_7$alkylene, $C_3$-$C_7$alkenylene or $C_3$-$C_7$alkynylene ; $R_1$ denote $C_2$-$C_8$alkanoyl, halogen-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl ; $R_2$ denotes $C_1$-$C_7$alkyl, phenyl- or naphthyl-$C_1$-$C_7$alkyl ; $R_3$ and $R_3'$ represent a common bond, or $R_3$ denotes hydrogen or $C_2$-$C_8$alkanoyl, halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkylsulfonyl or benzene- or naphthylsulfonyl, and $R_3'$ is hydrogen ; $R_4$ denotes hydrogen, $C_3$-$C_7$cycloalkyl, phenyl, biphenylyl or naphthyl ; $R_7$ denotes hydrogen or $C_1$-$C_7$alkyl ; where the aromatic radicals in each case are unsubstituted or substituted one or more times by substituents selected from the group consisting of halogen, $C_1$-$C_7$alkyl, $C_1$-$C_7$alkoxy, hydroxyl, $C_2$-$C_8$alkanoyloxy, trifuoromethyl and nitro, characterized in that correspondingly substituted starting materials are used.

4. Process according to claim 2 for the manufacture of a rifamycin SV derivative of the formula I or a salt thereof, in which $-A_1-A_2-$, $-A_3-A_4-$, $-A_5-A_6$ and $R_5$ have the indicated meanings; X represents $>C(R_6)-$ or $>N-$, and $R_6$ is hydrogen; alk denotes $C_1$-$C_7$alkylene, $C_3$-$C_7$alkenylene or $C_3$-$C_7$alkynylene ; $R_1$ and $R_3$, independently of one another, denote $C_2$-$C_8$alkanoyl, halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl, and $R_1$ additionally and $R_3'$ and $R_7$ denote hydrogen ; $R_2$ denotes $C_1$-$C_7$alkyl, phenyl- or naphthyl-$C_1$-$C_7$alkyl ; $R_4$ denotes hydrogen, $C_3$-$C_7$cycloalkyl, phenyl, biphenylyl or naphthyl ; where the aromatic radicals are in each case unsubstituted or substituted one or more times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro, characterized in that correspondingly substituted starting materials are used.

5. Process according to claim 2 for the manufacture of a rifamycin SV derivative of the formula I or a salt thereof, in which $-A_1-A_2-$, $-A_3-A_4-$, $-A_5-A_6-$ and $R_5$ have the indicated meanings; X represents $>C(R_6)-$ or $>N-$, and $R_6$ is hydrogen; alk denotes $C_1$-$C_7$alkylene, $C_3$-$C_7$alkenylene or $C_3$-$C_7$alkynylene, in particular $C_1$-$C_4$alkylene, $C_3$-$C_4$alkenylene or $C_3$-$C_4$alkynylene, where the multiple bonds are located in a position higher than $\alpha$ to the piperazine nitrogen; $R_1$ denotes $C_2$-$C_8$alkanoyl, phenyl-$C_2$-$C_6$alkanoyl, benzoyl, $C_1$-$C_7$alkoxy-carbonyl, phenyl-$C_1$-$C_4$alkoxycarbonyl, $C_1$-$C_4$alkanesulfonyl or benzenesulfonyl ; $R_2$ denotes $C_1$-$C_7$alkyl or phenyl-$C_1$-$C_7$alkyl ; $R_3$ and $R_3'$ together represent a bond or $R_3$ denotes hydrogen, $C_2$-$C_8$alkanoyl, phenyl-$C_2$-$C_6$alkanoyl, benzoyl, $C_1$-$C_7$alkoxy-carbonyl, phenyl-$C_1$-$C_4$alkoxycarbonyl, $C_1$-$C_4$alkanesulfonyl or benzenesulfonyl, and $R_3'$ is hydrogen; $R_4$ denotes hydrogen, $C_3$-$C_7$cycloalkyl, phenyl, biphenylyl or naphthyl ; $R_7$ denotes hydrogen or $C_1$-$C_7$alkyl; where the aromatic radicals in each case are unsubstituted or substituted up to three times by substituents selected from the group consisting of halogen, $C_1$-$C_7$alkyl, $C_1$-$C_7$alkoxy, hydroxyl, $C_2$-$C_8$alkanoyloxy, trifluoromethyl and nitro, characterized in that correspondingly substituted starting materials are used.

6. Process according to claim 2 for the manufacture of a rifamycin SV derivative of the formula I or a salt thereof, in which $-A_1-A_2-$, $-A_3-A_4-$, $-A_5-A_6-$ have the indicated meanings; X represents $>C(R_6)—$ or $>N—$, and $R_6$ is hydrogen; alk denotes $C_1$-$C_7$alkylene ; $R_1$ denotes $C_2$-$C_8$alkanoyl ; $R_2$ denotes $C_1$-$C_4$alkyl or phenyl-$C_1$-$C_4$alkyl, $R_3$ and $R_3'$ together represent a bond, or $R_3$ denotes hydrogen, $C_2$-$C_8$alkanoyl, benzoyl, $C_1$-$C_4$alkanesulfonyl or benzenesulfonyl, and $R_3'$ is hydrogen ; $R_4$ denotes hydrogen, $C_3$-$C_7$cycloalkyl, phenyl, biphenylyl or naphthyl, $R_5$ is acetyl ; $R_7$ is hydrogen or $C_1$-$C_4$alkyl ; where the aromatic radicals in each case are unsubstituted or substituted up to three times by substituents selected from the group consisting of halogen, $C_1$-$C_4$alkyl, $C_1$-$C_4$alkoxy, hydroxyl, $C_2$-$C_6$alkanoyloxy, trifluoromethyl and nitro, characterized in that correspondingly substituted starting materials are used.

7. Process according to claim 2 for the manufacture of a rifamycin SV derivative of the formula I or a salt thereof, in which $-A_1-A_2-$, $-A_3-A_4-$ and $-A_5-A_6-$ have the indicated meanings; X represents $>C(R_6)—$ or $>N—$, and $R_6$ is hydrogen; alk denotes $C_1$-$C_5$alkylene ; $R_1$ denotes branched $C_3$-$C_6$alkanoyl ; $R_2$ denotes $C_1$-$C_4$alkyl or phenyl-$C_1$-$C_4$alkyl ; $R_3$ denotes hydrogen, $C_2$-$C_6$alkanoyl, benzoyl or $C_1$-$C_4$alkanesulfonyl such as methanesulfonyl, and $R_3'$ is hydrogen ; $R_4$ denotes hydrogen, $C_3$-$C_7$cycloalkyl or phenyl which is unsubstituted or substituted by up to three $C_1$-$C_4$alkyl substituents ; $R_5$ is acetyl ; $R_7$ denotes hydrogen or $C_1$-$C_4$alkyl, characterized in that correspondingly substituted starting materials are used.

8. Process according to claim 2 for the manufacture of a rifamycin SV derivative of the formula I or a salt thereof, in which the structural elements $-A_1-A_2-$, $-A_3-A_4-$ and $-A_5-A_6-$ each have the indicated meanings; X represents $>N—$ ; $R_1$ denotes branched $C_3$-$C_8$alkanoyl ; $R_2$ denotes $C_1$-$C_4$alkyl ; $R_3$ denotes hydrogen or $C_2$-$C_5$alkanoyl ; $R_3'$ is hydrogen ; $R_5$ is acetyl ; alk-$R_4$ denotes $C_3$-$C_5$alkyl, $C_3$-$C_6$cycloalkyl-methyl or 2,4,6-tri-$C_1$-$C_4$-alkyl-benzyl ; $R_7$ is hydrogen, characterized in that correspondingly substituted starting materials are used.

9. Process according to claim 2 for the manufacture of a rifamycin SV derivative of the formula I or a pharmaceutically acceptable salts thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene; X represents $>CH—$ or $>N—$ ; alk denotes methylene or 2,2-dimethyl-1,3-propylene; $R_1$ denotes pivaloyl; $R_2$ denotes methyl, ethyl or benzyl ; $R_3$ and $R_3'$ represent a common bond, or $R_3$ denotes hydrogen or acetyl, and $R_3'$ is hydrogen ; $R_4$ denotes hydrogen, cyclohexyl or 2,4,6-trimethyl-phenyl ; $R_5$ denotes hydrogen or acetyl ; $R_7$ denotes hydrogen, characterized in that correspondingly substituted starting materials are used.

10. Process according to claim 2 for the manufacture of a rifamycin SV derivative of the formula I or a salt thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$ denotes vinylene; X represents $>N—$ ; $R_1$ denotes pivaloyl; $R_2$ denotes methyl or ethyl ; $R_3$ and $R_3'$ each denote hydrogen ; $R_5$ denotes acetyl ; $R_7$ is hydrogen ; and alk-$R_4$ denotes 2,4,6-trimethylbenzyl, characterized in that correspondingly substituted starting materials are used.

11. Process according to claim 2 for the manufacture of a rifamycin SV derivative of the formula I selected from

the group consisting of
4-O-methyl-8-O-pivaloyl-1-deoxy-15-deoxo-1,15-oxy-3-[4-(2,4,6-trimethylbenzyl)-piperazin-1-yl]-rifamycin SV ;
16,17,18,19-tetrahydro-4-O-methyl-8-O-pivaloyl-1-deoxy-15-deoxo-1,15-oxy-3-(4-(2,4,6-trimethylbenzyl)-piperazin-1-yl]-rifamycin SV ;
16,17,18,19,28,29-hexahydro-11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV ;
16,17,18,19,28,29-hexahydro-11-acetyloxy-4-O-methyl-8-O-pivaloyl-1-deoxy-11,15-dideoxo-1,15-oxy-3-[4-(2,4,6-trimethylbenzyl)-piperazin-1-yl]-rifamycin SV ;
4-O-ethyl-8-O-pivaloyl-1-deoxy-15-deoxo-1,15-oxy-3-[4-(2,4,6-trimethylbenzyl)-piperazin-1-yl]-rifamycin SV ;
4-O-benzyl-11-hydroxy-8-O-pivaloyl-1-deoxy-11,15-dideoxo-1,15-oxy-3-[4-(2,4,6-trimethylbenzyl)-piperazin-1-yl]-rifamycin SV ;
16,17,18,19,28,29-hexahydro-4-O-ethyl-11-hydroxy-8-O-pivaloyl-1-deoxy-11,15-dideoxo- 1,15-oxy-3-[4-(2,4,6-trimethylbenzyl)-piperazin-1-yl]-rifamycin SV ;
16,17,18,19,28,29-hexahydro-4-O-benzyl-11-hydroxy-8-O-pivaloyl-1-deoxy-11,15-deoxo-1,15-oxy-3-[4-(2,4,6-trimethylbenzyl)-piperazin-1-yl]-rifamycin SV ;
11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV ;
11-hydroxy-4-O-methyl-8-O-pivaloyl-1-deoxy-11,15-deoxo-1,15-oxy-3-[4-cyclohexylmethyl-piperazin-1-yl)-rifamycin SV ;
16,17,18,18,19,28,29-hexahydro-11-hydroxy-4-O-methyl-8-O-pivaloyl-1-deoxy-11,15-dideoxo-1,15-oxy-3-[4-cyclohexylmethyl-piperazin-1-yl)-rifamycin SV ;
16,17,18,19-tetrahydro-11-hydroxy-4-O-methyl-8-O-pivaloyl-1-deoxy-11,15-dideoxo-1,15-oxy-3-[4-(2,4,6-trimethylbenzyl)-piperazin-1-yl]-rifamycin SV ;
4-O-ethyl-11-hydroxy-8-O-pivaloyl-1-deoxy-11,15-dideoxo-1,15-oxy-3-(4-neopentyl-piperazin-1-yl)-rifamycin SV ;
11-hydroxy-4-O-methyl-8-O-pivaloyl-1-deoxy-11,15-dideoxo-1,15-oxy-3-(4-neopentyl-piperazin-1-yl)-rifamycin SV ;
11-hydroxy-4-O-methyl-8-O-pivaloyl-1-deoxy-11,15-dideoxo-1,15-oxy-3-(4-cyclohexylmethylpiperidin-1-yl)-rifamycin SV ;
4-O-methyl-8-O-pivaloyl-1-deoxy-15-deoxo-1,15-oxy-3-[4-(2,4,6-trimethylbenzyl)-piperazin-1-yl]-rifamycin SV ;
and the pharmaceutically acceptable salts thereof, characterized in that correspondingly substituted starting materials are used.